# EUROPEAN PATENT SPECIFICATION

(11) **EP 3 426 769 B1**
(45) Date of publication and mention of the grant of the patent: **25.09.2024**
(21) Application number: 17715816.9
(22) Date of filing: 09.03.2017
(51) Int. Cl.: C12N 5/071, B01L 3/00

(54) **DOUBLE TUBULAR STRUCTURES**
DOPPELRÖHRENSTRUKTUREN
STRUCTURES TUBULAIRES DOUBLES

(30) Priority: 09.03.2016 NL 2016404
(43) Date of publication of application: 16.01.2019
(73) Proprietor: Mimetas B.V., 2342 DH Oegstgeest (NL)
(72) Inventor: VULTO, Paul, 2342 DH Oegstgeest (NL); KUREK, Dorota Malgorzata, 2342 DH Oegstgeest (NL); JOORE, Adrianus Theodorus, 2342 DH Oegstgeest (NL); TRIETSCH, Sebastiaan Johannes, 2342 DH Oegstgeest (NL); LANZ, Henriëtte Leonore, 2342 DH Oegstgeest (NL)
(74) Representative: Algemeen Octrooi- en Merkenbureau B.V.
(86) International application number: PCT/NL2017/050145
(87) International publication number: WO 2017/155399

(56) References cited:
- WO-A1-2015/171074
- WO-A1-2017/066507
- KATELYN L. SELLGREN ET AL: "A biomimetic multicellular model of the airways using primary human cells", LAB ON A CHIP, vol. 14, no. 17, 1 January 2014 (2014-01-01), pages 3349 - 3358, XP055555540, ISSN: 1473-0197, DOI: 10.1039/C4LC00552J
- MARET BAUER ET AL: "3D microchannel co-culture: method and biological validation", INTEGRATIVE BIOLOGY, vol. 2, no. 7-8, 1 January 2010 (2010-01-01), UK, pages 371, XP055301187, ISSN: 1757-9694, DOI: 10.1039/c0ib00001a
- KYUNG EUN SUNG ET AL: "Transition to invasion in breast cancer: a microfluidic in vitro model enables examination of spatial and temporal effects", INTEGRATIVE BIOLOGY, vol. 3, no. 4, 1 January 2011 (2011-01-01), pages 439 - 450, XP055196312, ISSN: 1757-9694, DOI: 10.1039/C0IB00063A
- ERIC W. ESCH ET AL: "Organs-on-chips at the frontiers of drug discovery", NATURE REVIEWS. DRUG DISCOVERY, vol. 14, no. 4, 1 January 2015 (2015-01-01), GB, pages 248 - 260, XP055301188, ISSN: 1474-1776, DOI: 10.1038/nrd4539
- BHATIA SANGEETA N ET AL: "Microfluidic organs-on-chips", NATURE BIOTECHNOLOGY, vol. 32, no. 8, August 2014 (2014-08-01), pages 760 - 772, XP002761628
- YULI WANG ET AL: "Capture and 3D culture of colonic crypts and colonoids in a microarray platform", LAB ON A CHIP: MINIATURISATION FOR CHEMISTRY, PHYSICS, BIOLOGY, MATERIALS SCIENCE AND BIOENGINEERING, vol. 13, no. 23, 1 January 2013 (2013-01-01), GB, pages 4625, XP055300492, ISSN: 1473-0197, DOI: 10.1039/c3lc50813g
- NIHAL E. VRANA ET AL: "Engineering Functional Epithelium for Regenerative Medicine and In Vitro Organ Models: A Review", TISSUE ENGINEERING PART B-REVIEWS, vol. 19, no. 6, 1 December 2013 (2013-12-01), US, pages 529 - 543, XP055300465, ISSN: 1937-3368, DOI: 10.1089/ten.teb.2012.0603
- VINCENT VAN DUINEN ET AL: "Microfluidic 3D cell culture: from tools to tissue models", CURRENT OPINION IN BIOTECHNOLOGY., vol. 35, 19 June 2015 (2015-06-19), GB, pages 118 - 126, XP055258624, ISSN: 0958-1669, DOI: 10.1016/j.copbio.2015.05.002

## Description

### Background of the invention

Epithelium is specialized and polarized tissue that forms the lining of internal and external body surfaces. The cells forming the epithelium are closely packed and may form one or more layers. Epithelium may be one cell thick (simple epithelium) or two or more cells thick (stratified epithelium). Different types of epithelium, both simple and stratified, are recognized based on shape and function, including squamous epithelium, cuboidal epithelium, columnar epithelium, and transitional epithelium.

Normally a thin sheet of connective tissue, which is termed the basement membrane separates epithelium from underlying tissue. The basement membrane provides structural support for the epithelium and connects it to neighboring structures. The basement membrane acts as a scaffolding on which epithelium can grow and regenerate after injuries. Epithelial tissue is innervated, but avascular and epithelium must be nourished by substances diffusing from the blood vessels in the underlying tissue. The basement membrane acts as a selectively permeable membrane that determines which substances will be able to enter the epithelium
Differentiation of epithelium during development is closely associated with ordered sequence of morphogenetic events. Several experimental studies have emphasized that these developmental processes are dependent upon reciprocal epithelial - mesenchymal interactions.

There is a significant interest in the development of *in vitro* models of epithelial barrier tissues that replicate the organization and restrictive behavior observed *in vivo,* and which, for example will allow their use for non-invasive, rapid, economic, and reproducible testing and/or screening of new drug candidates, chemicals and foodstuffs. However, important signals are lost when cells are cultured ex vivo on two-dimensional plastic substrata. The obtained tissues in many cases do not exhibit the morphological characteristics of their *in vivo* equivalent tissue and many specialized differentiated cell types are absent.

Efforts to address these limitations led to the development of 3D cell-culture models in which cells are grown embedded in an extracellular matrix. This approach enhances expression of differentiated functions and improves tissue organization (Pampaloni et al. (2007). Nat Rev Mol Cell Biol 8: 839-84).

In particular, great recent progress has been made in the field of organoid culture. An organoid is a three-dimensional organ-bud that is typically comprised of most specialized cells that are also available in the human body. In practice, the culture and differentiation of tissue during embryonic development is mimicked in an in vitro environment, such that stem cells differentiate to various differentiated cells. A well-known example of such organoids are the small intestinal organoids (Shoichi Date and Toshiro Sato, Mini-Gut Organoids: Reconstitution of the Stem Cell Niche, Annu. Rev. Cell Dev. Biol., 2015, Vol. 31: 269-289). A cocktail of growth factors and signaling molecules such as Wnt pathway agonists (e.g. Wnt3a, R-spondin, CHIR99021), BMP/TGF pathway inhibitors (e.g. Noggin), EGF and an environment of basement membrane extract (matrigel or similar), assures culture of primary gut crypts, maintenance of its stem cell niche and potential of differentiation of cells towards for example goblet cells, enterocytes and enteroendocrine cells. This leads to a three-dimensional structure having secondary morphology aspects of the gut, including crypt and villus formation. Similar three-dimensional cultures have been established for the culture of primary human esophageal, gastric, colon, liver and pancreatic.

More recently, tremendous progress has been booked on growing brain organoids from induced pluripotent stem cells. Long term culture of suspended spheroids under continuous shaking lead to so-called minibrains with specialized sections such as fore- and hindbrain characteristics. Even more recently, a breakthrough has been realized in the culture of the kidney glomerulus, using a complex culture protocol, starting with induced pluripotent stem cells on transwell systems, that lead again to highly specialized cells that are present in the glomeruli of human kidneys.

A disadvantage of such organoid techniques is the lack of structural control over the mini-organs. Particularly independent apical-basal access is lacking due to the spheroidal shapes. It has been attempted to apply the organoid protocols to create flat polarized tissues on transwell membranes, such that apical-basal access is made possible but progress so far is highly limited, possibly, since an extracellular matrix context is important for the organoid growth, and incorporation of this does not yield leak-tight barriers.

Static *in vitro* models have been developed by culturing epithelial cells from different sources alone or in combination with supporting cells (feeder layers or mesenchymal cells like fibroblast) on a semipermeable membrane in the transwell setup. Unfortunately, these models exhibit low trans-epithelial electrical resistance (TEER), high permeability of typically impermeable marker molecules, low expression and functionality of transporters (e.g. the P-glycoprotein efflux pump), and short term viability. This may limits their value as a model.

Feeder layers are commonly used as a support of culture of many types of embryonic and adults stem cells. For example, mouse embryonic fibroblasts (MEFs) are frequently used to support culture of embryonic stem cells (ESCs). Maintenance of another stem cell type hematopoietic stem cells (HSCs) can be achieved and boosted by a co-culture with stromal mesenchymal stem cells.

Typically, feeder cells consist of a sheet of cells which are mitotically inactive and serve as substitute niche cells secreting the necessary growth factors and cytokines that are important in the maintenance of the desired phenotype of the target cell type. Feeder cells support the growth of other cells not only by releasing growth factors to the culture media, but also by providing extracellular matrix support, which enhance the desired cell-ECM interactions. The interaction of stem cell with its microenvironment regulates mechanism of self-renewal and differentiation capacity of stem cells. But as mentioned, in the Transwell setup, unfortunately, these models exhibit low trans-epithelial electrical resistance (TEER), high permeability of typically impermeable marker molecules, low expression and functionality of transporters (e.g. the P-glycoprotein efflux pump), and short term viability, which limit the value as a model, also in combination with feeder layers.

Maret Bauer et al. (Maret Bauer et al. Integrative Biology, 2010, vol. 2 no. 7-8, page 371) discloses a breast carcinoma model wherein epithelial carcinoma cells are co-cultured with mesenchymal fibroblast in a 3D micro-culture system. Kyung Eun Sung et al. (Kyung Eun Sung et al. Integrative Biology, 2011, vol. 14, no. 4, pages 248 - 260) describes a method for co-culturing human mammary fibroblasts and mammary epithelial cells in a microfluidic 3D system in the presence of collagen and shows the use of said system to analyse the transition of DCIS to IDC. WO2017/066507A1 discloses a method wherein a lung organoid in a microfluidic device is obtained by depositing an endothelial cell layer comprising mammalian endothelial cells on a first side of a membrane (e.g., porous membrane), depositing a stromal cell layer comprising mammalian lung fibroblast cells on a second side of the membrane that is opposite the first side of the membrane; and depositing an epithelial cell layer comprising mammalian lung epithelial cells. Katelyn L. Sellgren et al. Lab on a Chip ,2014, vol. 14, no. 17, pages 3349-3358 discloses a biomimetic microfluidic model of the human airway using all primary cells.

Also, the following prior art documents disclose artificial tissues cultured on microfluidic systems:
Eric w. Esch et al., Nature reviews. Drug discovery, vol. 14, no. 4, 1 January 2015 (2015-01-01), pages 248-260, WO2015/171074A1, Bhatia Sangeeta N et al. Nature biotechnology, vol. 32, no. 8, August 2014 (2014-08), pages 760-772, Yuli Wang et al., Lab on a chip: Miniaturisation for Chemistry, physics, biology, materials science and Bioengineering, vol. 13, no. 23, 1 January 2013 (2013-01-01), page 4625, Nihal e. Vrana et al., Tissue Engineering Part b-reviews, vol. 19, no. 6, 1 December 2013 (2013-12-01), pages 529-543

In addition, current methods and means do not allow high-throughput studies, such as analyses of absorption, transport and/or secretion, across an epithelial tissue. For example, known transwell plates are not suited for measuring absorption, transport and/or secretion across a sample of an epithelial tissue as the tissue sample will not sufficiently adhere to the membranes of the transwell plates.

Thus, there is need to develop a more defined and predictive model culturing human epithelia in which the proliferation and the differentiation of cells is mimicking the in vivo situation. In light of this, products, compositions, methods for and uses of improved *in vitro* epithelial models would be highly desirable, but are not yet readily available. In particular there is a clear need in the art for reliable, efficient and reproducible methods that allow to provide such *in vitro* epithelial barrier models with independent basal-apical access and that, for example may exhibit most specialized cells also present in the in-vivo equivalent tissue. These models may be used, for example, in high throughput screening, drug adsorption, transport and toxicity studies, disease modelling, interaction with e.g. microbial cultures and/or models for studying nutrient uptake. Accordingly, the technical problem underlying the present invention can been seen in the provision of such products, compositions, methods and uses for complying with any of the aforementioned needs. The technical problem is solved by the embodiments characterized in the claims and herein below.

### Summary of the invention

The current invention is set out in the appended set of claims.

### Description

### Drawings

Figure 1: Examples of a device for culturing an epithelial tube (not to scale): bottom view.

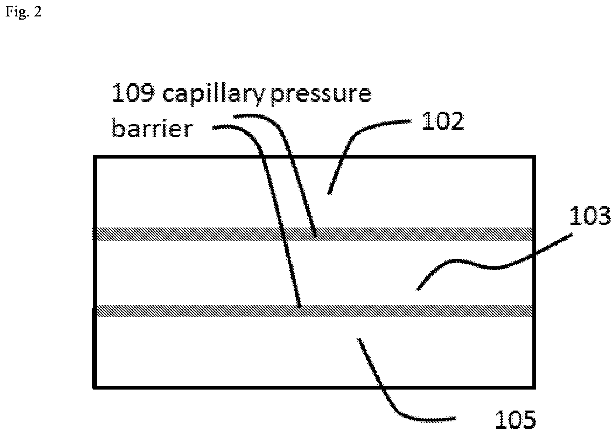

Figure 2: Examples of a device for culturing an epithelial tube (not to scale): close up of viewing window.

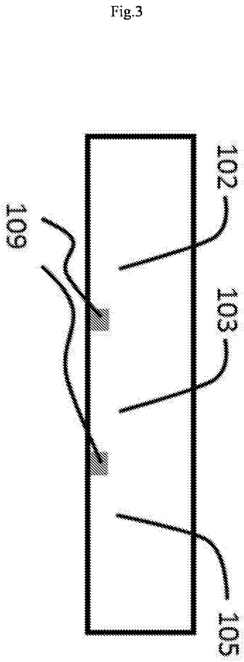

Figure 3: Examples of a device for culturing an epithelial tube (not to scale): vertical cross section of figure 2.

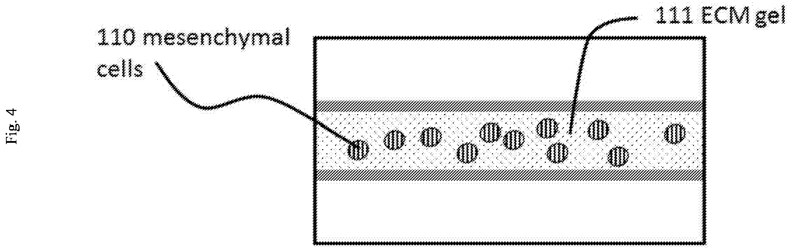

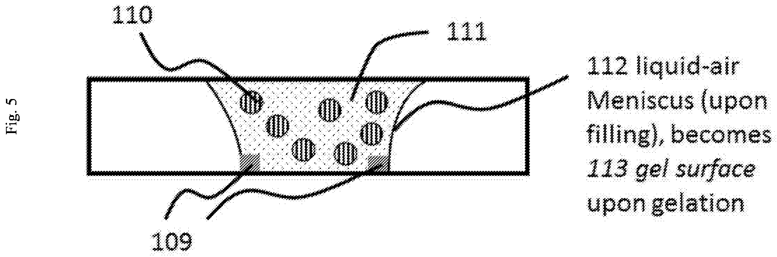

Figures 4 and 5: Step in a method for culturing an epithelial tube: an ECM gel precursor comprising a mesenchymal cells is inserted into the gel lane of figure 2/3, is pinned on the capillary pressure barrier and allowed to gelate. ECM may, for example, be Matrigel (either growth factor reduced or not), collagen I, collagen IV, fibrinogen, fibronectin, or combinations thereof as well as synthetic ECM.

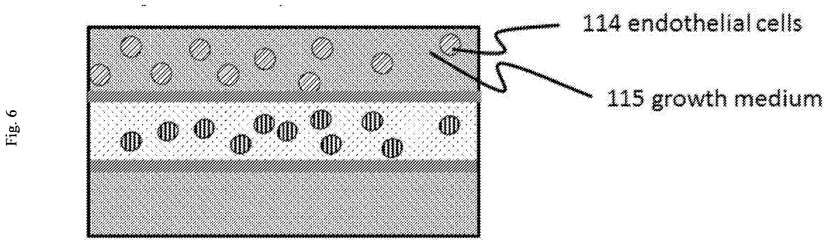

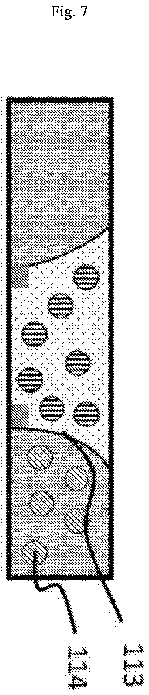

Figures 6 and 7: Step in a method for culturing an epithelial tube following the step described in figure 4/5 , wherein the epithelial cells are introduced into a first perfusion channel (and optionally growth medium is introduced in the second perfusion channel).

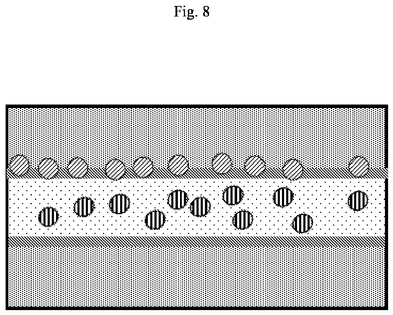

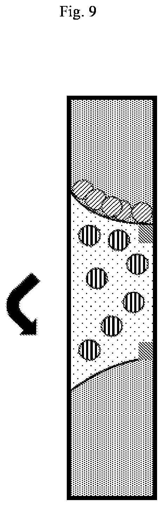

Figures 8 and 9: Step in a method for culturing an epithelial tube following the step described in figure 6/7, wherein the device of figure 3 is placed vertically such that epithelial cells are settling on the gel surface. Upon adhesion of epithelial cells a flow is induced (not shown).

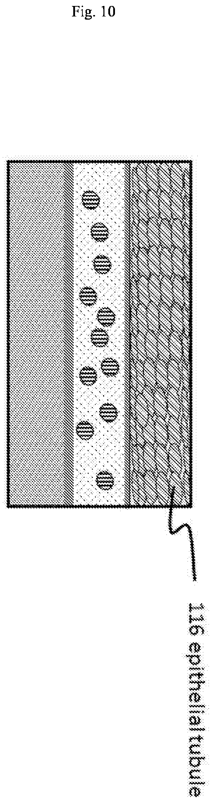

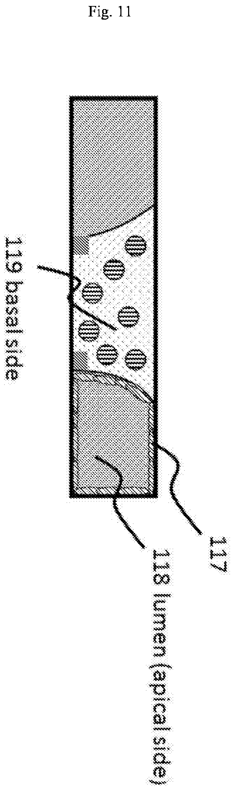

Figures 10 and 11: Step in a method for culturing an epithelial tube following the step described in figure 8/9, wherein the epithelial cells are allowed to proliferate and line channel walls and gel surface in order to form a tubule.

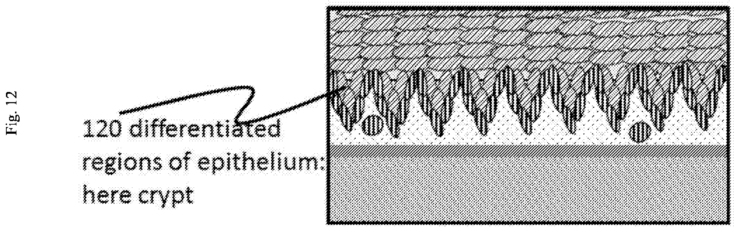

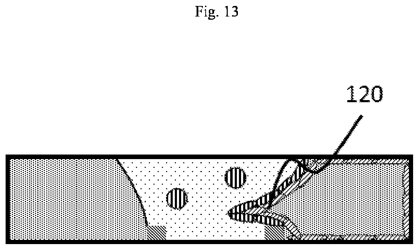

Figures 12 and 13: Step in a method for culturing an epithelial tube following the step described in figure 10/11, wherein the mesenchymal cells are allowed to interact with the epithelial cells and the epithelium is allowed to differentiate; differentiation may lead to a regular morphological pattern: here crypt structures.

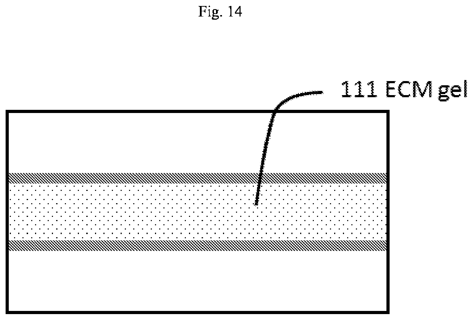

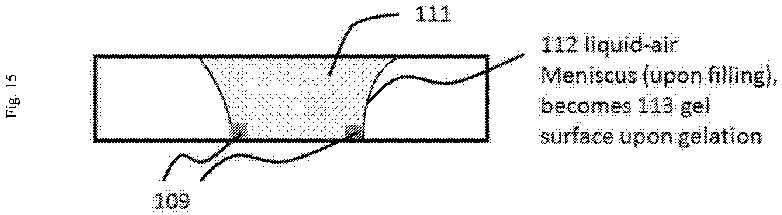

Figures 14 and 15: Step in a method for culturing an epithelial tube: an ECM gel precursor is inserted into the gel lane of figure 2/3, is pinned on the capillary pressure barrier and allowed to gelate.

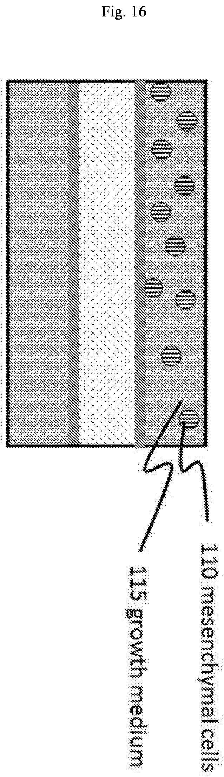

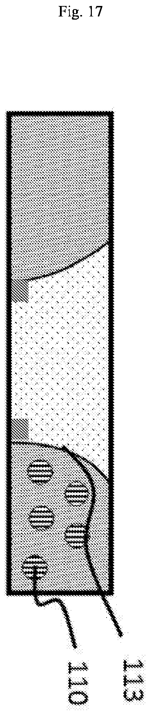

Figures 16 and 17: Step in a method for culturing an epithelial tube following the step described in figure 14/15 , wherein the mesenchymal cells are introduced into a first perfusion channel (and optionally growth medium is introduced in the second perfusion channel).

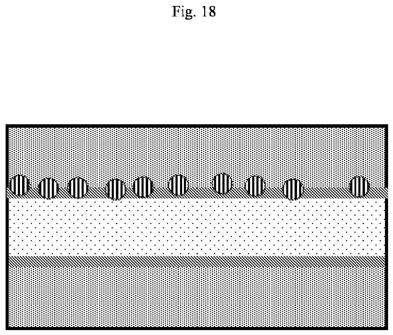

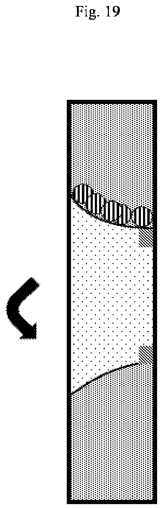

Figure 18 and 19: Step in a method for culturing an epithelial tube following the step described in figure 16/17, wherein the device of figure 3 is placed vertically such that mesenchymal cells are settling on the gel surface. Upon adhesion of mesenchymal cells a flow may be induced (not shown).

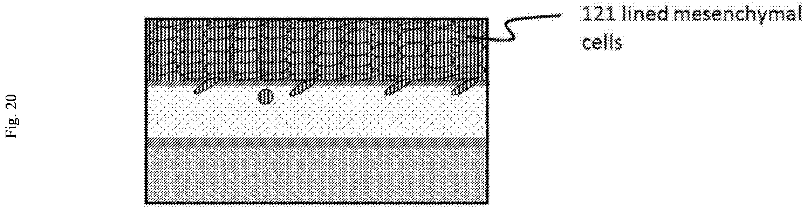

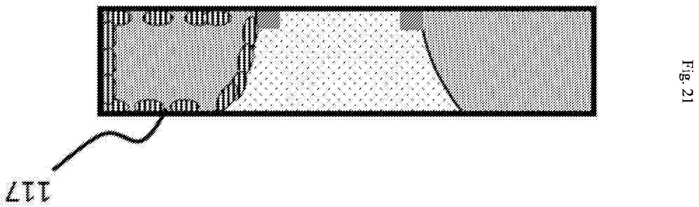

Figure 20 and 21: Step in a method for culturing an epithelial tube following the step described in figure 18/19 , wherein the mesenchymal cells are allowed to proliferate and line channel walls and gel surface.

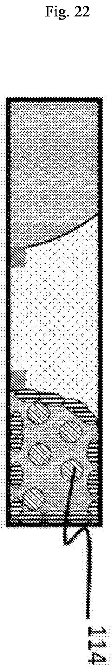

Figure 22: Step in a method for culturing an epithelial tube following the step described in figure 20/21, wherein the epithelial cells are introduced into a first perfusion channel (and optionally different medium is used).

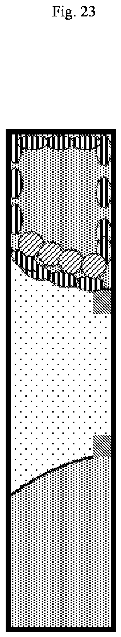

Figure 23: Step in a method for culturing an epithelial tube following the step described in figure 22, wherein the device of figure 3 is placed vertically such that epithelial cells are settling on the mesenchymal cells that are settled on the gel surface. Upon adhesion of mesenchymal cells a flow may be induced (not shown).

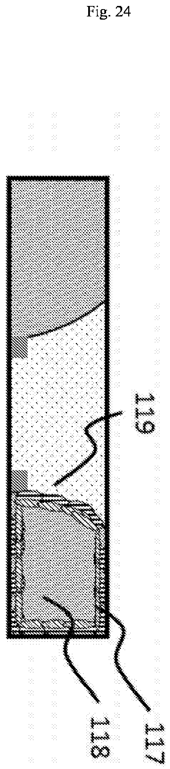

Figure 24: Step in a method for culturing an epithelial tube following the step described in figure 23 , wherein the epithelial cells are allowed to proliferate and line channel walls and gel surface in order to form a tubule having tight junctions.

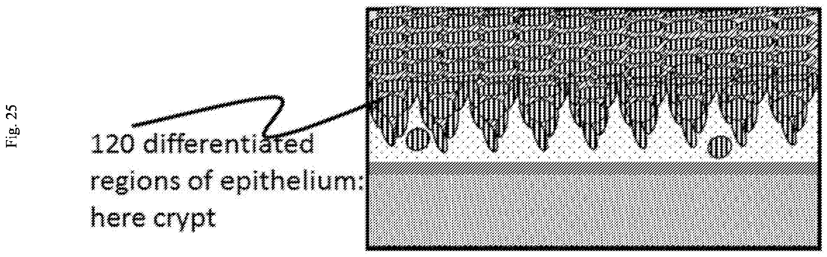

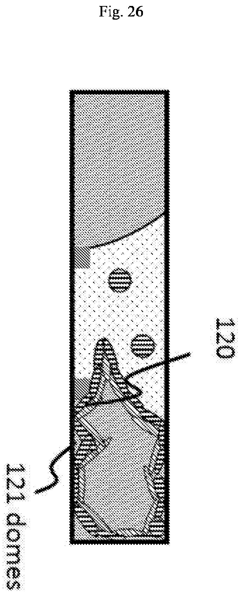

Figures 25 and 26: Step in a method for culturing an epithelial tube following the step described in figure 24, wherein the mesenchymal cells and the epithelial cells are allowed to interact and the epithelium is allowed to differentiate; differentiation may lead to a regular morphological pattern: here crypt structures and domes.

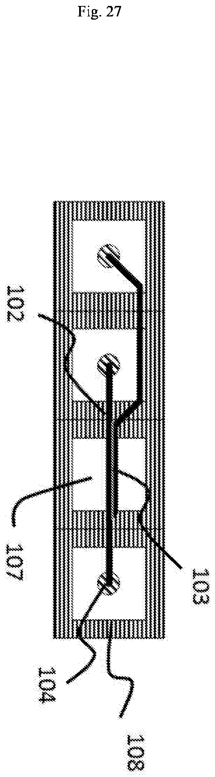

Figure 27: Alternative embodiment to figure 1 having 1 gel lane and one perfusion lane.

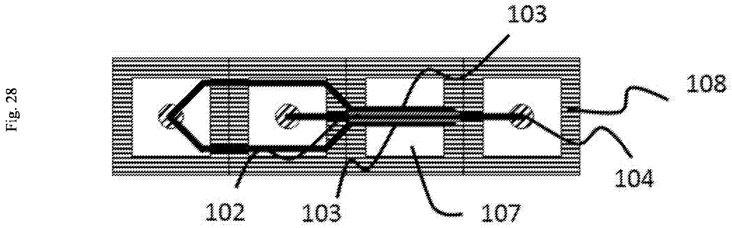

Figure 28: Alternative embodiment to figure 1 having 2 gel lanes that are filled from a single inlet (may optionally be separate inlets)
Figure 29: 1. Phase contrast images after sequential seeding of mesenchymal and epithelial cells in a 3-lane OrganoPlate^{®} (MIMETAS) with 400 micron wide lanes as shown in figure 1.

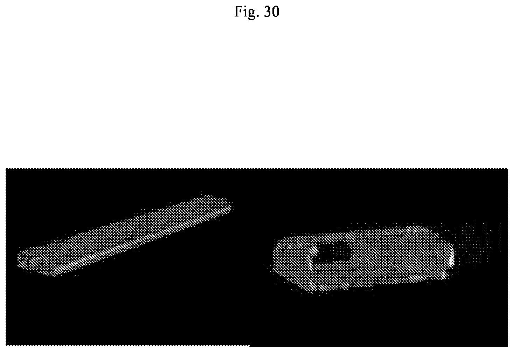

Figure 30: Confocal microscopy results after seeding of mesenchymal/epithelial cells in a 2-lane OrganoPlate^{®} (MIMETAS) with 400 micron wide lanes, showing tubular structure

### Definitions

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

"A," "an," and "the": these singular form terms include plural referents unless the content clearly dictates otherwise. Thus, for example, reference to "a cell" includes a combination of two or more cells, and the like.
"About" and "approximately": these terms, when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.
"Comprising": this term is construed as being inclusive and open ended, and not exclusive. Specifically, the term and variations thereof mean the specified features, steps or components are included. These terms are not to be interpreted to exclude the presence of other features, steps or components.
"Exemplary": this terms means "serving as an example, instance, or illustration," and should not be construed as excluding other configurations disclosed herein.

### Detailed Description

It is contemplated that any method, use or composition described herein can be implemented with respect to any other method, use or composition described herein. Embodiments discussed in the context of methods, use and/or compositions of the invention may be employed with respect to any other method, use or composition described herein. Thus, an embodiment pertaining to one method, use or composition may be applied to other methods, uses and compositions of the invention as well.

The inventors of the present invention have surprisingly found that the technical problem underlying the present invention may be solved by a method of microfluidic cell culturing as described herein.

Microfluidic cell culturing is an increasingly important technology. The technology finds its application in drug screening, tissue culturing, toxicity screening, and biologic research. A major advantage of microfluidic cell culturing is that it may add aspects such as perfusion flow, enhanced co-culturing and stable gradients to traditional cell culture, and may provide higher-quality data, reduced reagent consumption, and lower costs.

Numerous aspects related to microfluidic systems, devices, methods and manufacturing are discussed in the prior art, including patent documents such as WO 2008/079320, WO 2013/151616, WO 2010/086179, WO2012/120101, or as commercially available from, for example, Mimetas, Leiden, The Netherlands (e.g. OrganoPlate; www.mimetas.com). While no particular limitations should be read form those applications and documents into any claims presented herein, these documents provide useful background material related to specific embodiments.

High quality sample preparations are important for many clinical, research, and other applications. Culturing, characterization and visualization of cells has become increasingly valued in the fields of drug discovery, disease diagnoses and analysis, and a variety of other therapeutic and experimental work. It is of significant importance that with microfluidic cell culture technology in vitro samples may be obtained that closely represent their in vivo characteristics. Such in vitro samples may potentially benefit a wide range of molecular and cellular applications.

The technical problem underlying the present invention lies in the field of cell culturing methods and systems that are able to provide in vitro epithelial cell cultures that more closely represent their in vivo characteristics. This may including polarity (expression of apical and basolateral proteins, such as transporter and channel proteins (eg OAT2/3, MATE1/2, NKCC1), expression and functioning of structure-related proteins (e.g. villin in brush borders, actin), membrane receptors (e.g. EGFR/ErbB), adherens junctions, focal adhesions, morphology of cell and cell layer formation (shape and appearance; dimensions; microvilli, cilia, confluency) and function (barrier function, expression of cell surface receptors, uptake and secretion).

Most importantly these models preferably exhibit differentiation of cells into specialized cells in specific locations while preferably maintaining stem cell niches in other specific locations. Examples of such specialized cells in the small intestine comprise enterocytes, goblet cells, Paneth cells, in the kidney podocytes, various specialized cuboidal epithelia, in the retina retinal pigment epithelium, rods, cones, bipolar cells, ganglion cells, in the lung type I squamous alveolar cells, type II great alveolar cells. Differentiation of cells at specific locations, not only lead to specialized cells with distinct function and behaviors, but also in many cases changes the shape of the tissue, giving it its characteristic morphology. Examples are crypt-villi structures and mucin production in the small intestine, alveoli in the lung, glomerula, distal and proximal tubules and loops of Henle in the kidney, pigmental layer and layers of rods and cones in the retina. We refer to these characteristic shapes as *secondary morphology* in order to differentiate against *primary morphology,* such as flat pancake-like cell layers in transwell and surfaceattached cell cultures, or tubular structures of a tissue in the *in vivo* situation or in microfluidic systems.

Providing in vitro samples that better correspond to their in vivo counterparts is important.

In the art some methods using microfluidic cell culturing systems, microchambers or microfluidics have been proposed. Most other systems use standard culture plates and use various barrier inserts in an attempt to culture epithelial cells that more closely represent their in vivo characteristics (e.g. Transwell permeable supports). Currently available systems, however, have not yet fulfilled both with regard to providing in vitro epithelial cell samples closely resembling in vivo characteristics and with regard to a number of aspects necessary for ease-of-use, high-throughput, or automated applications.

The inventors of the present invention have surprisingly found that the problems in the art can be solved by providing a method of culturing and/or monitoring epithelial cells using a microfluidic cell culture system comprising a microfluidic channel network.

The method of the present invention allows for tube-formation in a microfluidic device that may display secondary morphology and provides specialized, polarized and, differentiated cells. This may be according to a pattern that seems to resemble tissue organization *in vivo.* This is achieved, in short, by lining of epithelial cells with mesenchymal cells and, in a preferred embodiment, the use of gel, e,g, an extracellular matrix gel, that further accommodates the secondary morphology, in contrast to those methods in the art, for example, transwell systems, that use a rigid structure.

Therefore, according to a first aspect of the present invention there is provided a method of culturing and/or monitoring epithelial cells using a microfluidic cell culture system comprising a microfluidic channel network wherein the method comprises
a) introducing mesenchymal cells in the microfluidic channel network, wherein the mesenchymal cells are introduced in the microfluidic channel network
   a1) using an aqueous medium; or
   a2) using a gel precursor and allowing the gel precursor to gelate in the microfluidic channel network thereby occupying at least part of the microfluidic channel network;
b) in case of step a1), and preferably in case of step a2), allowing the mesenchymal cells to proliferate and/or differentiate until at least part of the microfluidic channel network is covered with mesenchymal cells such that at least a group/layer/sheet of mesenchymal cells is formed;
c) introducing epithelial cells in the microfluidic channel network comprising the mesenchymal cells; and
d) allowing the epithelial cells to proliferate and/or differentiate, until at least part of the microfluidic channel network surface (wall) is covered with epithelial cells and/or until at least part of the mesenchymal cells is covered with epithelial cells such that at least a group/layer/sheet of epithelial cells is formed.

Alternatively, there is provided a method of culturing and/or monitoring epithelial cells using a microfluidic cell culture system comprising a microfluidic channel network wherein the method comprises
A1.1) introducing a gel precursor, preferably an extracellular matrix gel precursor in the microfluidic channel network, e.g. in part of the microfluidic channel network, e.g. in a hollow volume.
A1.2) allowing the gel to set or gelate;
A1.3) introducing mesenchymal cells in another art of the microfluidic channel network that is not covered by the ECM gel; or
A2) mixing the cells with a gel precursor and allowing the gel precursor to gelate in the microfluidic channel network thereby occupying at least part of the microfluidic channel network;
B) in case of step a1), and preferably in case of step a2), allowing the mesenchymal cells to proliferate and/or differentiate, preferably until at least part of the microfluidic channel network is covered with mesenchymal cells;
C) introducing epithelial cells in the microfluidic channel network comprising the mesenchymal cells; and
D) allowing the epithelial cells to proliferate and/or differentiate, preferably until at least part of the microfluidic channel network surface is covered with epithelial cells and/or until at least part of the mesenchymal cells is covered with epithelial cells.

Whereas in the description and claims reference will be made to first method described above (with e.g. steps a) - d) , the skilled person understand that that any method, use or composition described herein can likewise be implemented with respect to the method presented with alternative wording (with e.g. steps A) - D)).

In the method of the present invention, in a microfluidic channel network, cells of mesenchymal origin are cultivated to form a first group of cells forming a layer or sheet. After the mesenchymal cells were allowed to cover at least part of the surfaces of microfluidic network and/or the gel, epithelial cells are introduced in the microfluidic channel network, preferably within the layer or sheet of mesenchymal cells (i.e. away from the (artificial) wall of the microfluidic channel). The epithelial cells (and the mesenchymal cells) are allowed to proliferate and/or differentiate, preferably at least until confluency is reached.

With the method of the invention, a layer of epithelial cells is provided that is in direct contact with a layer of mesenchymal cells, possibly with an intermediate basal lamina equivalent that is excreted by the two cell types and that is more resembles the in vivo situation is comparison to methods described in the art. For example, the mesenchymal cells and/or basal lamina can be in direct contact with the epithelial cells, without the presence of any artificial, non-natural or from the outside introduced membrane, such as the membranes used in transwell systems. At the same time, with the method of the present invention, the epithelial cells have reduced contact with the artificial (e.g. plastic or glass) wall (surface) of the microfluidic channel network of the microfluidic cell culture system

In addition, by the use of a extracellular matrix gel the secondary morphology of the cells is further accommodated, in contrast to those methods in the art that use, for example, transwell systems providing a rigid structure of at least 10µm of an artificial porous membrane.

Without being bound by theory, the present inventors speculate that proliferation and differentiation of the cells depends on bidirectional communication between the epithelial cells and the mesenchymal cells and that this communication is improved by the method of the invention, particularly due to the absence of such rigid structures as applied in the transwell systems, and/or by preventing or reducing contact of the epithelial cells with the rigid walls of the culturing device employed and/or by creating, in the hollow microfluidic channel (ie in the microfluidic channel network) a (micro)environment allowing proliferation and differentiation of the cells more resembling the in vivo situation.

Not wishing to be bound by any specific theory, we hypothesize that the presence of mesenchymal cells are instructive towards the epithelium. The exchange of signaling molecules, e.g. morphogens, specifically results in patterns of combinations of such molecules, e.g. morphogens. A specific combination of these at a specific location may result in the maintenance of the stem cell niche, while another combination of morphogens at another specific location results in the differentiation towards specific subtypes. A morphogen is generally understood as a substance governing the pattern of tissue development in the process of morphogenesis, and the positions of the various specialized cell types within a tissue. More specifically, a morphogen may be a signaling molecule that acts directly on cells to produce specific cellular responses depending on its local concentration.

Not wishing to be bound to any specific theory we hypothesize that specific combinations of signaling molecules, morphogens in particular, occur at more or less regular intervals. Regular should here be interpreted in the context of biology, that is a regularity such as the stripes of a zebra, or the patches on a panter's fur: not a precise regularity, but a clear pattern.

Morphogens that are crucial in such pattern formation include, but are not limited to Wnt-family members, hedgehog family members, noggin, bone morphogenic protein, epithelial growth factors (EGF), fibroblast growth factors (FGF) and Dickkopf (DKK) proteins.

The extracellular matrix or basal lamina is an important element in the formation of such regular patterns, as it may bind certain morphogenic factors, resulting in a local concentration, while allowing others to diffuse.

Within the context of the current invention, a microfluidic network is a hollow volume defined by two side walls (surfaces), a bottom substrate, and a top substrate closing the channel network. Both side walls, top substrate and bottom substrate can be referred to as walls when being in contact with the microfluidic channel network. The channel network is furthermore connected to an inlet, typically a hole in the top substrate, that is used to fill the network from the outside world. Furthermore a vent needs to be present that upon filling the network with a first fluid (typically a liquid), allows expulsion of the fluid that is present in the network (typically air). The channel network may comprise one microfluidic channel or multiple microfluidic channels that are connected to one another. The microfluidic channel network can also be connected to further inlets or outlets.

In a first step of the method, mesenchymal cells are introduced in the microfluidic channel network of the microfluidic cell culture system.

The mesenchymal cells that may be used in the present invention may be any type of cells of mesenchymal origin. The mesenchymal cell or at least one or more mesenchymal cells include fibroblasts, myofibroblasts, smooth muscle cells, adipocytes, chondroblasts, osteoblasts and stromal cells from different regions of the body including the bone marrow, prostate, heart, lung, gut, kidney, blood vessels and tendons. Preferably, the mesenchymal cells are fibroblasts or myofibroblasts. The mesenchymal cells may be in a proliferative state or mitotically inactive. The mesenchymal cells may be differentiated mesenchymal cells or mesenchymal progenitor cells. By mesenchymal progenitor cell is meant a multipotent cell of mesenchymal origin, e.g. a cell capable of differentiating into various lineages of mesenchymal origin. For the avoidance of doubt, with mesenchymal cells we intend cells of mesenchymal origin.

The mesenchymal cells may be neonatal or adult cells. Preferably, the mesenchymal cells are mammalian cells, more preferably human mesenchymal cells. The mesenchymal cells can be freshly isolated cells or multiple passaged cells. The mesenchymal cells may be primary cells or a (immortalized) cell line. The mesenchymal cells may be isolated from healthy or disease tissues, including tumors. The mesenchymal cells may comprise more than one type of mesenchymal cell. Mesenchymal cells may also be obtained through stem cell techniques, such as induced pluripotent stem cell techniques. Mesenchymal cells may also be derived from epithelia, through induction of epithelial to mesenchymal transition (EMT).

In a preferred embodiment, the mesenchymal cells are selected from myofibroblasts, fibroblasts, adipocytes, chondroblasts, osteoblasts, smooth muscle cells and stromal cells, preferably wherein the mesenchymal cells are mammalian cells, preferably human cells.

The cells may be introduced in the microfluidic channel network by any suitable means. For example, the cells may be introduced using an aqueous medium, typically cell culture medium. Cell culture media must be able to deliver all the nutrients and other compounds that are essential for the growth and/or proliferation of the cells, but they preferably may not contain compounds that could be harmful to the growth and/or proliferation of the cells.

The cells may be dispersed in said medium and introduced in the microfluidic channel by allowing the medium to enter the microfluidic channel network. Typically a pipette may be used to dispense cells in medium in an inlet and allowing the microfluidic channel network to fill through capillary force. Alternatively, cells in medium may be introduced into the microfluidic channel network through active pumping. It will be understood by the skilled person that once the cells are introduced in the microfluidic channel network, the cells should be allowed to settle and to start differentiating and/or proliferating. Settling of the cells could be onto one of the surfaces Preferably the aqueous medium used is a medium suitable for proliferation and/or differentiation of the mesenchymal cells. Compositions of such media are widely known in the art and any suitable growth medium, if so desired supplemented with additional (growth) factors, may be used. After the cells settled and optionally attached to the walls of (if present) the gel, e.g. the extracellular matrix gel and/or the microfluidic channel network, suitable growth medium that provides nutrients and oxygen to the mesenchymal cell is provided, allowing the mesenchymal cells to proliferate and/or differentiate. The growth medium may be provided in a flow or not. In the case of a flow, the growth medium may also remove or dilute waste metabolites as produced by the cells.

Alternatively, the mesenchymal cells may be introduced in the microfluidic channel network using a gel precursor. The cells may be dispersed/suspended in said gel precursor and introduced in the microfluidic channel network by allowing the gel precursor to enter the microfluidic channel network, and allowing to fil selected regions of the network with help of patterning techniques such as for example capillary pressure barriers. Subsequently the gel precursor is allowed to gelate (solidify) in certain regions of the microfluidic channel thereby occupying at least part of the microfluidic channel network. With respect to the term occupation of at least part of the microfluidic channel network, it will be understood by the skilled person that it is not required that gel is present throughout the microfluidic channel network, but preferably occupying certain areas or the network, such that selected other regions remain accessible for introducing a further gel or a growth medium for e.g. a perfusion flow. It will also be understood that the gel should not block passage of growth medium through the microfluidic channel network.

The gel precursor can be provided to the channel as described above. After the gel is provided, it is caused to gelate, prior to introduction of a further fluid. Suitable (precursor) gels are well known in the art. By way of example, the gel precursor, may be a hydrogel, and is typically an extracellular matrix (ECM) gel. The ECM may for example comprise collagen, fibrinogen, fibronectin, and/or basement membrane extracts such as Matrigel or a synthetic gel. The gel precursor may, by way of example, be introduced into an inlet with a pipette (typically a repeating pipette such as the Eppendorf Multipette^{®} M4 (Eppendorf AG, Germany, catalogue number 4982 000.012) in combination with Eppendorf Combitips advanced ^{®} (Eppendorf AG, Germany, catalogue number 0030 089.405).

The gel may thus comprise a basement membrane extract, human or animal tissue or cell culture-derived extracellular matrices, animal tissue-derived extracellular matrices, synthetic extracellular matrices, hydrogels, collagen, soft agar, egg white and commercially available products such as Matrigel.

Basement membranes, comprising the basal lamina, are thin extracellular matrices which underlie epithelial cells in vivo and are comprised of extracellular matrices, such a protein and proteoglycans. Although an epithelial cell layer, multilayer or monolayer, prevents the invasion of an exogenous material from the external world as a barrier, a basement membrane itself also acts as a physical barrier. Thus, epithelial cells comprising an epithelial tissue collaborate with a basement membrane to form a solid barrier and to protect the internal vital activity.

They are composed of collagen IV, laminin, entactin, heparan sulfide proteoglycans and numerous other minor components (Quaranta et al, Curr. Opin. Cell Biol. 6, 674-681, 1994). These components alone as well as the intact basement membranes are biologically active and promote cell adhesion, migration and, in many cases growth and differentiation. An example of a gel based on basement membranes is termed Matrigel (US 4829000). This material is very biologically active in vitro as a substratum for epithelial cells.

Many different suitable gels for use in the method of the invention are commercially available, and include but are not limited to those comprising Matrigel rgf, BME1, BME1rgf, BME2, BME2rgf, BME3 (all Matrigel variants) Collagen I, Collagen IV, mixtures of Collagen I and IV, or mixtures of Collagen I and IV, and Collagen II and III), puramatrix, hydrogels, Cell-Tak^{™},Collagen I, Collagen IV, Matrigel^{®} Matrix, Fibronectin, Gelatin, Laminin, Osteopontin, Poly-Lysine (PDL, PLL), PDL/LM and PLO/LM, PuraMatrix^{®} or Vitronectin. In one preferred embodiment, the matrix components are obtained as the commercially available Corning^{®} MATRIGEL^{®} Matrix (Corning, NY 14831, USA).

MATRIGEL^{®} Matrix is extracted from the Engelbreth-Holm-Swarm ("EHS") mouse tumor, a tumor rich in basement membrane. The major matrix components are laminin, collagen IV, entactin, and heparin sulfate proteoglycan ("HSPG"). The matrix also contains growth factors, matrix metalloproteinases (collegenases), and other proteinases (plasminogen activators), as well as some as yet undefined extracellular matrix components. At room temperature, MATRIGEL^{®} Matrix gels to form a reconstituted basement membrane.

Preferably, the gel (precursor) is a basement membrane extract, an extracellular matrix component, collagen, collagen I, collagen IV, fibronectin, laminin, vitronectin, D-lysine, entactin, heparan sulfide proteoglycans or combinations thereof. /pct
The gel precursor is released into the inlet of and is transported into the microfluidic network by capillary forces, potentially assisted by gravity. The gel may, again by way of example, be halted, for example with a phaseguide, which is essentially a capillary pressure barrier that spans the complete width of the microfluidic channel network and caused to gelate. After the gel is formed, a suitable growth medium that provides nutrients and oxygen to the mesenchymal cell in the gel is provided, allowing the mesenchymal cells to proliferate and/or differentiate. The growth medium may be provided in a flow or not. In the case of a flow, the growth medium may also remove or dilute waste metabolites as produced by the cells.

Patterning of the gel precursor, e.g. ECM gel precursos, can be done in a variety of ways including, photolithograpihic patterning and patterning with capillary pressure techniques. The function and patterning of capillary barriers have been previously described by the applicants, e.g. in WO2014038943. The capillary pressure barriers are not to be understood as a wall or a cavity which is filled with the gel precursor, but consists of elements which make sure that the gel precursor due to the surface tension does not spread open. This concept is referred to as meniscus pinning. As such, stable confinement of fluid meniscii consisting of (ECM) gel precursor will be achieved in the microfluidic channel.

The capillary pressure barrier provided could for example consist of a rim of material protruding out from the bottom substrate, or a groove protruding into the bottom substrate. The sidewall of the rim having an angle with the top of the rim that is preferably as large as possible. In order to provide a good barrier, this angle needs to be larger than 70°, typically around 90°. The same counts for the angle between the sidewall of the ridge and the top-side of the bottom substrate.

An alternative manner for creating the capillary pressure barrier is to apply a line of material on the bottom substrate that is significantly more hydrophobic than the surrounding material. The latter acts as a spreading stop due to capillary force/surface tension. As a result, the liquid is prevented from flowing beyond the capillary pressure barrier and enables the formation of stable confined meniscus in the microfluidic channel network. Thus in particular embodiments, the capillary pressure barriers used are in particular selected from a rim, a groove, a hole, or a hydrophobic line of material or combinations thereof. In another embodiment capillary pressure barriers can be created by pillars at selected intervals that are lining the area that is to be occupied by the gel.

Alternative manner of selectively patterning an (ECM) gel precursor include the use of a sacrificial layer or removable structure that is present in the microfluidic channel network upon inserting the gel precursor and is removed upon gelation of the gel.

Alternatively a photosensitive cross-linker may be present in the gel, such that upon exposure to e.g. UV light, the gel gelates. Masking the light source enables selective gelation of the gel precursor and allows to remove non-solidified gel precursor from those regions that should be devoid of the gel.

After the mesenchymal cells are introduced in the microfluidic channel network, either using an aqueous medium, preferably a growth medium, or by using the gel (precursor), the mesenchymal cells are allowed to proliferate and/or differentiate in the microfluidic channel network. Proliferation of the mesenchymal cells is continued for a period until at least part of the microfluidic channel network is covered with mesenchymal cells. Upon bringing the cells in culture in the microfluidic channel, they typically form a tubular structure that can be perfused with a flow through the lumen of the tubular structure (i.e. that side of the cells that is faced away from the wall of the channel).

In other words, once the mesenchymal cells are introduced in the microfluidic channel network, the mesenchymal cells are allowed to grow, differentiate, expand and divide in order to allow the cells to form in the microfluidic channel network a sheet, layer, group, of cells.

In embodiments wherein no gel is present in the microfluidic channel network, the cells may form a sheet, layer of group of cells that is at least partially attached to the (rigid) wall of the channel.

In some embodiments, and that will be detailed below, part of the microfluidic channel network comprises a gel, wherein the gel precursor was not provided with mesenchymal cells, and wherein, for example the mesenchymal cells are introduced in the channel using an aqueous medium. In such embodiments, the mesenchymal cells may form a group or sheet or layer of cells on the gel present in the microfluidic channel network, as well as on the (rigid) wall of the channel not formed by the gel (e.g. the plastic or glass wall of the microfluidic channel network, depending on what type of material the wall is made of).

In embodiments wherein the mesenchymal cells are introduced in the channel using a gel precursor, the cells are allowed to grow, divide, proliferate and/or differentiate in the gel, and/or to grow outside the gel, into the microfluidic channel network.

With respect to the covering of the microfluidic channel network, this encompasses the presence of mesenchymal cells in the gel only, in the channel only and both in the gel and in the channel. In a preferred embodiment, the mesenchymal cells cover the whole of the area of the microfluidic channel were the cells were introduced (and may thus form a tubular structure). This may be referred to as 100 percent confluency. Confluence is the term commonly used as an estimate of the number of adherent cells in the microfluidic device, referring to the proportion of the surface which is covered by cells. For example, 50 percent confluence means that roughly half of the surface is covered. When a layer is said to be confluent, about 100 percent of the surface of the gel is covered by the cells, and no more room is left for the cells to grow as a monolayer.

100 percent confluency, or covering of the microfluidic channel network (in the area wherein the cells are introduced, or are monitored) is not necessary, and a lower percent of coverage, by way of example 10, 20, 30, 40, 50, 60, 70, 80, or 90 percent, may suitable be used in the present invention. For example, the mesenchymal cells may be present in the gel only. In this latter case, mesenchymal cells do not necessarily or preferably grown on the channel walls, but preferably inside the (ECM) gel as clusters of cells.

As will be understood by those skilled in the art, in embodiments wherein the mesenchymal cells are introduced in the microfluidic channel network by means of a gel precursor, it is not necessary to allow the mesenchymal cells to proliferate and/or differentiate before introduction of the epithelial cells in the next step of the method of the present invention. It is also possible to introduce the epithelial cells in the channel after the gelation of the gel comprising the mesenchymal cells, and allow the mesenchymal and epithelial cells to proliferate and/or differentiate together.

However, preferably the mesenchymal cells are allowed to proliferate and differentiate before the epithelial cells are introduced in the culture system. The length of the period is dependent on various factors like the type of mesenchymal cell introduced, the method of introduction, the number of cells introduced, the composition of the growth medium used to proliferate and/or differentiate the cells, the temperature, and so on. For example, the period may be at least 20 minutes, at least one hour, at least 6 hours, at least 12 hours, at least 24 hours, at least one, two, three or four days. Typically this period is no longer than 14 days. Those skilled in the art will have no problem establishing those cultivation conditions suitable for use in the present invention.

Next, epithelial cells are introduced in the microfluidic channel network wherein the mesenchymal cells are present.

The epithelial cells that may be used in the present invention may be any type of cell of with epithelial characteristics, or capable of differentiating into a cells having these characteristics. Typically epithelial cells are of ectodermal or endodermal origin. When mentioning epithelial cells we also intent progenitor cells and stem cells with capability to differentiate towards epithelial cells as subject to the invention.

The epithelial cell or one or more epithelial cells may be a simple epithelium, such as simple squamous epithelium, such as mesothelium or endothelium. Alternatively, an epithelial cells may be a stratified epithelia, such as an epidermal cell or columnar epithelia cell. Such cells may include epithelial cells of kidney, colon, small intestine, lung, retina . The epithelial cells may be differentiated epithelial cells or epithelial progenitor cells. By epithelial progenitor cell is meant a multipotent cell having epithelial potential, e.g. a cell capable of differentiating into an epithelial cell.

The epithelial cells may be neonatal or adult cells. Preferably, the epithelial cells are mammalian cells, more preferably human epithelial cells. The epithelial cells can be freshly isolated cells or multiple passaged cells. The epithelial cells may be primary cells or a (immortalized) cell line. The epithelial cells may be isolated from healthy or disease tissues. The epithelial cells may comprise more than one type of epithelial cell. In some embodiments, two or more types of the epithelial cell are mixed at different ratios and allowed to grow on the mesenchymal cells.

Preferably the epithelial cells are selected from simple epithelia cells, simple squamous epithelia cells, stratified epithelia cells, or columnar epithelia cells, preferably wherein the epithelial cells are mammalian cells, preferably human cells.

In a preferred embodiment the epithelial cells used in the method of the invention are obtained from an in vitro cultivated organoid, for example as described in US2012/0196312. The cells in the organoid may, before introduction in the microfluidic channel network be treated to provide, for example single cells or clumps of cells (e.g. of 2 - 50 cells, preferably no more than 20, 10 cells).

In some embodiments, the epithelial cell and the mesenchymal cell have the same origin, i.e. are from the same type of animal or are from the same animal. Preferably the epithelial cells and the mesenchymal cells are from the same body part.

In some embodiments, the epithelial cell and the mesenchymal cell are from different origins, i.e. are from different types of animals, or are from different body parts of the same type of animal or of the same animal. In some embodiments, the epithelial cell is from a diseased tissue and the mesenchymal cell is from a healthy tissue. In some embodiments, the mesenchymal cells are from a diseased tissue and the epithelial cells are from a healthy tissue. In some embodiments, the cells are obtained from a tumor.

Also provided is that in step a) different types of mesenchymal cells are introduced and/or wherein in step c) different types of epithelial cells are introduced in the same microfluidic channel. This allows for the study of more complex epithelial systems, for example allows to study the interaction between different type of epithelial cells, of between epithelial cells form healthy and diseased tissues.

The epithelial cells may be introduced in the microfluidic channel network by any suitable means. Preferably, the cells may be introduced using an aqueous medium. The cells may be dispersed in said medium and introduced in the microfluidic channel by allowing the medium to enter the microfluidic channel network comprising the mesenchymal cell. It will be understood by the skilled person that once the cells are introduced in the microfluidic channel, the cells should be allowed to settle and to start proliferating. Preferably the aqueous medium used is a medium suitable for proliferation of the epithelial cells, and preferably of the epithelial and the mesenchymal cells. Compositions of such media are widely known in the art and any suitable growth medium, if so desired supplemented with additional (growth) factors, may be used. After the epithelial cells settled and attached, suitable growth medium that provides nutrients and oxygen to the cells is provided, allowing the epithelial cells (and the mesenchymal cells) to proliferate and/or differentiate. The growth medium may be provided in a flow or not. In the case of a flow, the growth medium may also remove or dilute waste metabolites as produced by the cells.

After the epithelial cells are introduced in the microfluidic channel network comprising the mesenchymal cells, the cells are allowed to proliferate and/or differentiate in the microfluidic channel network. Upon bringing the cells in culture in the microfluidic channel, they typically, form a tubular structure that can be perfused with a flow through the lumen of the tubular structure (i.e. that side of the cell layer that is faced away from the wall of the channel).

With tubular structure is meant that cells are lining most of the channel surfaces of the perfusion flow channel that are not covered by the ECM gel as well as the surface of the ECM gel itself that is facing the perfusion flow channel in which the epithelial cell suspension is introduced. The tubular structure typically forms along the complete length of the channel from one inlet to another. The inlet furthermore allows access to the inside or lumen of the tubule. In case of a flow of medium, the flow is applied to the luminal side of the epithelial tubule. Typically this coincides with the apical side of the epithelium.

Proliferation of the epithelial cells is continued for a period until at least part of the biological material formed by, and including the introduced mesenchymal cells, is covered by the biological material formed by, and including the introduced epithelial cells. In other words, the mesenchymal cells and the epithelial cells are cultivated for a period that allows the formation of a layer of epithelial cells that is in close contact with the mesenchymal cells, including any basal lamina or basal lamina like structure formed during the cultivation of the mesenchymal and epithelial cells. For example, the period may be at least 20 minutes, at least one hour, at least 6 hours, at least 12 hours, at least 24 hours, at least one, two, three or four days. Typically the period is for at least 6 hours, at least 22 hours, or at least one, two three or four days. Normally the period is no more than 14 days.

With respect to the covering of the mesenchymal cells, in a preferred embodiment, the epithelial cells cover the whole of the area that is covered by the mesenchymal cells. However, 100 percent coverage of the mesenchymal cells in the microfluidic channel network (in the area wherein the cells are introduced, or are monitored) is not necessary, and a lower percent of coverage, by way of example 10, 20, 30, 40, 50, 60, 70, 80, or 90 percent, may suitable be used in the present invention. However, in the present invention, at least part of the epithelial cells must be in close contact with at least part of the mesenchymal cells and/or any basal lamina and/or basal lamina like structure formed during the cultivation of the cells.

By way of example, the mesenchymal cells may only be present on the surface of a gel that is present in the microfluidic channel, and/or in and on a surface of a gel in those embodiments wherein the mesenchymal cells were introduced in the channel by means of a gel precursor, as detailed herein. Epithelial cells are to be understood to cover at least part of the mesenchymal cells when at least part of the area with the mesenchymal cell on or close to the surface of the gel is covered by the epithelial cells.

Detailed below, and in a highly preferred embodiment, the mesenchymal cells form a tubular structure in the microfluidic channel network, and within which tubular structure the epithelial cells are allowed to proliferate, thereby, in a preferred embodiment, forming a tubular structure within said tubular structure of mesenchymal cell, wherein the mesenchymal cells are at least partially covered by the epithelial cells. In such an embodiment, again, the mesenchymal cells and/or any basal lamina and or basal lamina-like structure formed during the cultivation of the cells, are in close contact with the epithelial cells.

It was found that with the present invention, the epithelial cells, and/or the mesenchymal cells more closely resemble epithelial and/or mesenchymal cells found in vivo, for example when compared to some methods in the prior art. This may be manifested by the cells by the expression of certain genes typical for the in vivo situation, by a morphology that more closely resembles in vivo morphology, by improved epithelial barrier function, by the presence and function of an apical and basolateral membrane, or even by the presence of villi, crypts, ciliated tissue, mucous membrane layer, and/or the presence of differently differentiated cells in the sheet or layer of epithelial cells. The epithelial cell layer may be secreting and/or absorbing different types of material in and from the medium. Most importantly, cells may be differentiating into various lineages of the tissue of origin.

Within the method of the present invention, it is also possible to introduce a gel precursor in the microfluidic channel network and allowing the gelprecursor to gelate in the microfluidic channel network thereby occupying at least part of the microfluidic channel network. In some embodiment, the gel precursor may comprise the mesenchymal cells, as described above, however it is also contemplated that a gel is introduced in the microfluidic channel network that does not comprise mesenchymal cells.

By way of example, a gel precursor may be introduced in the channel and allowed to gelate before the mesenchymal cells are introduced in the microfluidic channel network, for example by means of an aqueous medium.

In these embodiments, the walls of the microfluidic channel network are in part formed by the gel. Again, the gel precursor that is introduced may or may not comprise mesenchymal cells.

In case a gel is introduced without mesenchymal cells present therein, the mesenchymal cells may be introduced in the channel using the aqueous medium, preferably a growth medium that provides nutrients and oxygen. Via this medium, cells can be introduced in the channel thereby depositing them against the gel and allowing the mesenchymal cells to form a sheet, group or layer of cells, for example on the gel.

As stated before, upon bringing cells in culture in the microfluidic channel, they typically, but not necessarily, form a tubular structure that can be perfused with a flow through the lumen of the tubular structure (i.e. the side of the cell that is faced from the wall of the channel). Thus, in some embodiments, a gel is first provided to the channel such that after gelation, the mesenchymal cells can be introduced in the channel by means of a medium, for example a culture medium, allowing the cells to contact the gel and to form on the gel a layer of cells (e.g. a sheet, or tubular structure or vessel). Next, the epithelial cells can be introduced in the channel by means of a medium, for example a culture medium, allowing the epithelial cells to contact the mesenchymal cells and to form on the mesenchymal cells a layer of cells (e.g. a sheet, or tubular structure or vessel), thereby creating an apical and basolateral side.

Tubular structures goes by means of saying as it is not expected that cells of mesenchymal origin form a tight layer as is the case for an epithelium. Whereas epithelia are known to from tight junctions, have a coblestone shape with brush borders and villi, cells of mesenchymal origin, fibroblasts and myofibroblasts form a loose network without tight junctions. Epithelium expresses epithelial cell markers such as E-cadherin and villin, whereas mesenchymal cells express mesenchymal cell markers such as α-SMA and vimentin.

Both in embodiments wherein the gel precursor is used to introduce the mesenchymal cell and in embodiment wherein the gel precursor is not used to introduce the mesenchymal cells, multiple gels could be patterned adjacent one another. Multiple gels can be patterned by injecting gel precursors, halting advancement of the precursors by a capillary pressure barrier and causing the precursors to gelate in different parts of the network (channel) sequentially or in parallel. Suspension of a first cell type in a first gel precursor, followed by a second cell type in a second gel precursor results in a so-called stratified co-culture, in which cell types are cultured adjacent to one another. The gel preferably is in contact with/deposited against one or more channel walls.

Gels are defined as a substantially dilute cross-linked system, which remain in place once gelated, but allow for interstitial flow through the gel. A gel is often a non-fluid colloidal network or polymer network that is expanded throughout its whole volume by a fluid. A hydrogel, or aqua gel, is a gel in which the swelling agent is water. Within the context of the method of the invention, the gel material may be a water-containing gel that is preferably insoluble in water but comprises water so as to have a two- or three-dimensional support structure. In the present invention, the gel used allows for diffusion of a substance in and over said gel.

The gel used in the invention is not particularly limited as far as the layer has the above properties and allows for the forming of a layer of cells on the gel. Commonly used gels include gels from biological origin comprising collagen, laminin, fibronectin, fibrinogen, Matrigel and/or agarose, and synthetic gels based on several scaffolds such as PEG (polyethylene glycols), peptides, PLLA (poly-L-lactide), PLGA (poly(lactic-co-glycolic acid).

Several techniques can be used to pattern the gel, i.e.to fill part of the microfluidic channel with the gel, including but not limited to lithographic patterning of photocurable gels, capillary force based patterning using e.g. pillars, hydrophobic patches or phaseguides, and selective deposition.

Preferably the gel is patterned, preferably by use of a capillary pressure barrier, by UV patterning, or by retracting a needle after gelation, or by having a sacrificial layer that is removed after gelation.

As detailed above, the mesenchymal cells introduced in step a) may be dispersed/suspended in the gelprecursor or maybe introduced in the microfluidic channel network using an aqueous medium, preferably, and when a gel (e.g. a gel wherein no mesenchymal cells are dispersed) is present alongside the gel.

In the method of the present invention, in step b) the mesenchymal cells are proliferated until at least a group/layer/sheet of mesenchymal cells is formed in the microfluidic channel network and/or in the gel.

Mesenchymal cells cultivated in the method of the present invention may form a sheet or layer of cells. Such sheet or layer may be a monolayer but may also consist of more than one layer, and display different thickness along the sheet. The sheet or layer may be of any size.

Preferably in step b) the mesenchymal cells are proliferated until at least a tubular structure of mesenchymal cells is formed in the microfluidic channel network. Within the context of the present invention a tubular structure of mesenchymal cells is a structure formed by the cells growing from inlet to outlet of the microfluidic channel network, thereby lining the majority of channel and/or gel surfaces. Those skilled in the art understand that the structure does not need to be fully "round" tube, but may in fact have any form, for example as dictated by the form of the wall of the microfluidic channel network and/or the gel. However, the tubular structure does not necessarily has to follow the form of the channel but may adapt any type of α-regular of regular from, including a, by way of example, a circular or more rectangular formed tube.

It is preferred that the mesenchymal cells form a tubular structure as defined within the context of the invention as this allows the epithelial cells to be introduced within said tubular structure and to cover, in a tubular fashion, the mesenchymal cells. Such "tube-in-a-tube" or double tube tissue was found to closely resemble in vivo tissue with respect to phenotypical characteristics, such as those disclosed herein.

As for the mesenchymal cells, likewise, in step d) the epithelial cells are proliferated until at least a group/layer/sheet of epithelial cells is formed in the microfluidic channel network. The skilled person understands that the epithelial cells may cover part of the microfluidic channel network , e.g. wall or surface, including any gel if present, not covered by mesenchymal cells, but also part of the mesenchymal cells will be covered by the epithelial cells. Epithelial cells cultivated in the method of the present invention may form a sheet or layer of cells that is, depending on the type of epithelial cell used, either a monolayer, or formed of different layers (e.g. as may occur when a cells of a stratified epithelial tissue are used). The layer may display different thickness along the sheet. The sheet or layer may be of any size.

Preferably in step d) the epithelial cells are proliferated until at least a tubular structure of epithelial cells is formed in the microfluidic channel network. Within the context of the present invention a tubular structure of epithelial cells is a structure formed by the cells growing from inlet to outlet of the microfluidic channel network, thereby lining the majority of channel and/or gel surfaces either covered or not covered by the mesenchymal cells. Those skilled in the art understand that the structure does not need to be fully "round" tube, but may in fact have any form, for example as dictated by the form of the wall of the microfluidic channel network and/or the gel and/or by the form of the mesenchymal cells). However, the tubular structure does not necessarily has to follow the form of the channel or the form of the sheet of mesenchymal cells but may adapt any type of a-regular of regular from, including a, by way of example, a circular or more rectangular formed tube.

If the mesenchymal cells are introduced in step a) in a gel (ie. introduced using a gel precursor) it is preferred that in step d) of the method, the epithelial cells are proliferated until at least a group/layer/sheet of epithelial cells covers at least part of the gel that occupies at least part of the microfluidic channel network.

However, preferably both the mesenchymal cells and the epithelial cells form a tubular structure within the context of the present invention, whereby the epithelial cell layer is characterized by tight junction formation and the mesenchymal cell layer by a loose network of cells. Thus a method of the present invention is provided wherein in step d) the epithelial cells form a tubular structure inside a tubular structure that is formed by the mesenchymal cells. Also in this embodiment, the mesenchymal cells are at least in part covered by the epithelial cells, or, said otherwise, the epithelial cells are lined, at least partially by the mesenchymal cells. It is speculated that due to the close contact of the mesenchymal cells and the epithelial cells, communication between the cells, e.g. by secretable factors or signaling molecules such as members of the wnt family, hedgehog family (sonic hedgehog, indian hedgehog), noggin, BMP's, rspondin, notch-family and others, is optimized in comparison to for example methods employing transwell systems or comprising other types of supports, filters or membranes.

Under circumstance it may be preferred that the growth medium in the hollow microfluidic channel (ie in the microfluidic channel network) sample does not flow, or does flow, wherein said flow is uni-directional or bi-directional. In particular in case a tubular structure is obtained of either the mesenchymal cells or the epithelial cells, or, preferably, both, it may be preferred to apply a flow of growth medium through the lumen of the tubular structure.

By way of example, applying such flow may further trigger the epithelial cells to adopt a phenotype resembling in the in vivo situation, e.g. when also in the in vivo situation flow of liquid is applied to the epithelial cells.

Another example, the flow may be used to introduce or remove substance in the medium, e.g. drugs to be tested for their influence of epithelial functioning or reaction.

The skilled person understands that the growth medium used in the method of the invention is not particularly limited with respect to its composition. Depending on the circumstances, for example, of the cells used, it may be desirable to supplement the growth medium with certain factors (signaling molecules, growth factors, inhibitors and/or activators of signaling pathways) like Wnt, noggin, egf/fgf, notch ligands and/or Rspondin and other described herein. These factors are known to be instructive for maintaining the stem cell niche of epithelia, which in turn is important for proliferation and differentiation of pedigree cells into sub-lineages of the epithelia of interest. E.g. for the case of small intestinal organoids it was found that adding these factors to cells suspended in matrigel yields intact crypt-villi structures consisting of stem cells, enterocytes, goblet cells, paneth cells, enteroendocrine cells.

One of more factors may be provided at the stage of cultivating the mesenchymal cells, and/or at the stage of cultivating both the mesenchymal cells and the epithelial cells.

The one or more factors may be present throughout the cultivation of the cells or only for a limited period of time (e.g. for 1 - 24 hours, 48 hours, 72 hours, 1,2,3,4,5,6,7 or more days).

The one of more factors may be presented to the cells from the apical side of the epithelial cells or from the basolateral side of the epithelial cells, or from both sides.

The one or more factors may be an inhibitor or an activator of one or more of the signaling pathways described herein (e.g. hedgehog signaling pathways, Wnt signaling pathways, BMP signaling pathways). It is also contemplated that first the cells are treated with an inhibitor of a certain signaling pathway, and subsequently treated with an activator of the same pathway, or the other way around. It is also contemplated that the cells are treated on the apical side with an activator and on the basolateral side with an inhibitor of the same pathway, or the other way around. One or more factors may be used at the same time.

It is also contemplated that a concentration gradient of one or more factors is applied e.g. from the apical to the basolateral side, or along the hollow channel from inlet to outlet. The gradient may be linear or non-linear. The concentration of the factor may change depending on the stage of cultivation.
The factors may be supplied using the growth medium or via the gel, e.g. be dispersed in the gel before cultivation or be provided to the gel during cultivation.

With respect to the factors any combination of one, two, three, four or more, targeting one, two, three of more signaling pathways may be used.

Non-limiting, but preferred factors, to be targeted signaling pathways, inhibitors and activators thereof (e.g. factors) include:
- Activators and inhibitors of bone morphogenetic protein (BMP). BMPs constitute a group of pivotal morphogenetic signals, orchestrating tissue architecture throughout the body.

Example of suitable BMP signaling inhibitors include but are not limited to molecules involved in inhibition of the BMP signaling that is mediated by binding of BMP (bone morphogenetic protein) to a BMP receptor, including inhibitors such as Noggin (Noggin, also known as NOG, is a protein that is involved in the development of many body tissues, including nerve tissue, muscles, and bones; e.g. at a concentration of 10 - 500 ng/ml), chordin, and follistatin. Other examples of a small molecule BMP inhibitor having such properties include a compound that inhibits BMP2, BMP4, BMP6 or BMP7 capable of activating a transcription factor SMAD1, SMAD5, or SMAD8, such as Dorsomorphin (P. B. Yu et al. (2007), Circulation, 116: 11 60; RB. Yu et al. (2008), Nat. Chem. Biol., 4: 33-41; J. Hao et al. (2008), PLoS ONE (www. plozone. org), 3 (8): e2904). In addition examples of a BMP I-type receptor kinase inhibitor include LDN- 193189 (that is, 4-(6-(4-(piperazin-l-yl)phenyl)pyrazolo[l,5-a]pyrimidin-3-yl)quinolone; Yu PB et al. Nat Med, 14: 1363-9, 2008). LDN-193189 is commercially available from Stemgent, for example.

Examples of suitable BMP signaling activators include BMP (belonging to the transforming growth factor-beta (TGFB) superfamily; such as BMP1, BMP2, BMP4, BMP7, amongst others (for example, in concentration of between 0,1 ng/ml - 250 ng/ml medium.
- Activators and inhibitors of Wnt signaling. The Wnt signaling pathways are a group of signal transduction pathways made of proteins that pass signals into a cell through cell surface receptors. Three Wnt signaling pathways have been characterized: the canonical Wnt pathway, the noncanonical planar cell polarity pathway, and the noncanonical Wnt/calcium pathway. All three pathways are activated by binding a Wnt-protein ligand to a Frizzled family receptor, which passes the biological signal to the protein dishevelled inside the cell Wnt comprises a diverse family of secreted lipid-modified signaling glycoproteins that are 350-400 amino acids in length. The type of lipid modification that occurs on these proteins is palmitoylation of cysteines in a conserved pattern of 23-24 cysteine residues.

Examples of suitable Wnt activators include, but are not limited to BML-284; 2-Amino-4-[3,4-(methylenedioxy)benzylamino]-6-(3-methoxyphenyl)pyrimidine or DKK1 inhibitor; (1-(4-(Naphthalen-2-yl)pyrimidin-2-yl)piperidin-4-yl)methanamine, and proteins of the R-spondin family, including R-spondin-1 (e.g. at concentrations of 0,01 - 5 microgram/ml medium) and proteins of the Wingless-Type MMTV Integration Site Family, including Wnt3a and others (e.g. in a concentration of at least 50, 100, 500, 1000 ng/ml, e.g. between 50 - 1000 ng/ml).

Examples of Wnt signalling inhibitors include XAV-939, the PORCN inhibitor Wnt-C59 (C59), LGK-974, ICG-001, IWP-2, IWP-L6 and many others.
- Also suitable are GSKbeta inhibitors and/or activators. Glycogen synthase kinase-3 (GSK-3) is a proline-directed serine-threonine kinase that was initially identified as a phosphorylating and an inactivating agent of glycogen synthase. Two isoforms, alpha (GSK3A) and beta, show a high degree of amino acid homology. GSK3B is involved in energy metabolism, neuronal cell development, and body pattern formation.

Non-limiting examples of GSKbeta inhibitors include CHIR-99021 (CT99021), SB216763, CHIR-98014, Tideglusib, acetoxime, and AZD2858, LiCl (e.g. at a concentration of 0,1 mM - 100 mM). CHIR 99021 or CHIR 98014 may, for example, be used at a concentration of at least about 1 µM to about 20 µM in the medium.
- Another example is Epidermal growth factor or EGF, which is a growth factor that stimulates cell growth, proliferation, and differentiation by binding to its receptor EGFR. Human EGF is a 6045-Da protein with 53 amino acid residues. EGF may be used, for example, at concentration of 5 - 200 ng/nl, preferably 10 - 100 ng/ml, for example 50 ng/ml.
- Activators and inhibitor of the Notch pathway. The Notch signaling pathway is a highly conserved cell signaling system present in most multicellular organisms. Mammals possess four different notch receptors, referred to as NOTCH1, NOTCH2, NOTCH3, and NOTCH4. The notch receptor is a singlepass transmembrane receptor protein. Notch signaling promotes proliferative signaling during neurogenesis, and its activity is inhibited by Numb to promote neural differentiation. The Notch signaling pathway is important for cell-cell communication, which involves gene regulation mechanisms that control multiple cell differentiation processes during embryonic and adult life. Example of Notch pathway modulators include gamma-secretase inhibitors such as DAPT (e.g. in concentration of 0,1 - 50 micorM), and/or FLI-06, LY411575, Dibenzazepine, Semagacestat, L658, and others.
- Another example of Fibroblast growth factors, or FGFs, which are a family of growth factors, with members involved in angiogenesis, wound healing, embryonic development and various endocrine signaling pathways. The FGFs are heparin-binding proteins and interactions with cell-surface-associated heparan sulfate proteoglycans have been shown to be essential for FGF signal transduction. FGFs are key players in the processes of proliferation and differentiation of wide variety of cells and tissues.
- A further example of such factor are transforming growth factor beta (TGF-β), which is a multifunctional cytokine belonging to the TGF-β superfamily that includes three different isoforms (TGF-β 1-3) and many other signaling proteins.
- Endothelin-1
- PDGF-B and PDGFA, Platelet-derived growth factor subunit B and subunit A. The members of this family are mitogenic factors for cells of mesenchymal origin and are characterized by a motif of eight cysteines.
- Activators and inhibitors of Hedgehog signalling, including hedgehog proteins. The Hedgehog signaling pathway is a signaling pathway that transmits information to cells required for proper development. Mammals have three Hedgehog homologues, DHH, IHH, and SHH, of which Sonic (SHH) is the best studied. Suitable protein factors for use in the current invention include Shh, Ihh and Hh, for example in concentrations of 0,01 - 10 mg/ml, preferably 0,1 - 1 mg/ml, or lower). Inhibitors includey LDE 225, saridegib, BMS 833923, LEQ 506, PF- 04449913 and TAK-441.

These factors are known to the skilled person, and he knows how to use these within the context of the current invention.

Recently it was shown that the use of feeder layers of mesenchymal origin (in this case mitotically inactivated 3T3 fibroblasts) enabled growth of organoids on flat transwell substrates, without use of matrigel (X. Wang, Y. Yamamoto, L.H. Wilson, T. Zhang, B.E. Howitt, M.A. Farrow, F. Kern, G. Ning, Y. Hong, C.C. Khor, et al., Nature, 522 (2015), pp. 173-178). Also here it appeared possible to differentiate in the essential sub types of the small intestine. However, the rigid substrate of the transwell, did not allow for free generation of secondary morphology and the current inventors stipulate that differentiation is restricted because of this as well as absence of flow conditions.

With the method of the invention it is possible to cultivate epithelial cells in the presence of an mesenchymal feeder layer against, in a preferred embodiment, an gel, e.g. an extracellular matrix gel, thus providing full flexibility for formation of secondary morphology, in addition to growing tubular structures with clear apical/basal orientation and with the possibility of being perfused. For those cells being in contact with the gel there is full absence of a (rigid) wall or filter (e.g. a woven filter).

As detailed above, it is preferred that the epithelial sheet or tubular structure is lined by the mesenchymal cells, and wherein the mesenchymal cells are positioned between the walls of the microfluidic channel network and the epithelial cells. In other words, also provides is that at least part of the mesenchymal cells is positioned between the microfluidic channel network wall and the epithelial cells.

Also provided is that in step d) the epithelial cells are allowed to form a layer of cells with an apical and a basolateral side, the basolateral side being faced towards the mesenchymal cells. Important for apical-basal polarization is the presence of an ECM/Basal lamina. Also the use of perfusion flow yields nicely polarized tubules.

The apical membrane of a polarized cell is the surface of the plasma membrane that faces inward to the lumen. The basolateral membrane of a polarized cell is the surface of the plasma membrane that forms its basal and lateral surfaces. In vivo, it faces towards the interstitium, and away from the lumen. In the present invention, the basolateral membrane is the membrane that faces, or is in close contact with the mesenchymal cell(s) and or the gel, e.g. extracellular matrix gel. Epithelial cells form tight junctions with one-another, yielding a closely knit membrane. Each plasma membrane domain has a distinct protein composition, including specific transporters that allow for transport of certain compounds over the membrane either in basal or apical direction.

As mention above, the at least part of the mesenchymal cells are in close (or direct) contact with the least part of the epithelia cells. Within the context of the present invention, this is to indicate that the epithelial cells and the mesenchymal cells are connected to each other either directly or via the presence of a basal lamina that is formed between the cells during cultivation according to the present invention. Typically, the distance between the mesenchymal cell sheet and the epithelial cell sheet is a thickness or less than the thickness of a basal lamina (for example, preferably less than 100 micrometers, more preferably in the range of 10 micrometers). The skilled person understands that a basal lamina is the structural and functional interface between epithelial cells and, within the context of the present invention, the mesenchymal cells, important in growth and control mechanisms of the epithelial cells. The thickness of a basal lamina may vary, depending on e.g. the type or location of the epithelium, and the condition of the body, and may have thickness with values of, e.g. 30 - 300 nm, e.g. 100 nm (see, e.g. Dockery et al. Hum.Repr.Update (1998) 4(5):486-495), values smaller than the membranes and filters used in the art.

Also provided is that the method further comprises subjecting the epithelial cells to air by removal of aqueous medium present in the microfluidic channel network comprising the epithelial cells. Subjection to air may be performed after the mesenchymal cell and epithelial cells were allowed to proliferate, preferably forming a tubular structure. This embodiment is in particular preferred when using epithelial cells that under in vivo conditions, would also be subjected to air, for example in the lungs, skin or gut.

The skilled person understand that the epithelial cells may be subjected to a wide variety of conditions not limited to air, but that may include subjection to other gases, to fluids, to drugs and compounds, to food components. It is even contemplated that the cells are subject to bacteria, for example in the lumen of gastro-intestinal tract or vaginal epithelia.

Also provided is that the microfluidic cell culture system comprises a culture chamber, wherein the mesenchymal cells in step a) and the epithelial cells in step c) are introduced. Such chamber thus forms the microfluidic channel network).

In a preferred embodiment, the microfluidic channel network wherein the cells are introduced is characterized by the presence of a first part constructed to provide a fluid path to the cells and/or a second part constructed to provide a fluid path from said cells, preferably to and from the culture chamber comprising the mesenchymal cells and the epithelial cells. This allows for flow of growth medium through the channel and along the cells present in the channel, for example in the culture chamber.

With respect to the gel, when a gel is present, the gel may be provided in the microfluidic channel network, or in a channel adjacent to the microfluidic channel network, and wherein said gel is in direct contact with said microfluidic channel network. In both cases, the gel thus cover or forms part of the wall of the microfluidic channel network wherein the cells are introduced.

It may even be the case that, adjacent to the gel a further microfluidic channel network is present that is in contact with the gel but wherein said channel is not in direct contact with the microfluidic channel comprising the epithelial cells. For example, in case the gel is present in a channel that is adjacent to the channel wherein the cells will be introduced, the gel thus forms part of the wall of this channel. On the other side of the gel, a further channel may be present, and that may, for example be used to provide the gel with nutrients or compounds, or that may be used to collect materials secreted by the cells. Alternatively, the gel may be present on two sides of the perfusion channel. This embodiment has the advantage that the maximum gel surface is exposed towards the tubule. The gel may be introduced from several inlets or one common inlet. Particularly when working with capillary pressure barriers such as phaseguides, the meniscus of the gel precursor upon meniscus pinning is stretching into the perfusion channel, such that in cross section an arc-shaped meniscus is present. This may be advantageous to achieve a more spherical cross-section of the tubule to-be formed.

Also provided in that in the method of the present invention, the microfluidic cell culture system provides an uninterrupted optical path to the cells in the microfluidic channel network and/or to the gel and/or to the further microfluidic channel network. This will allow for the uninterrupted measurement, monitoring or observing of the cells cultivated in the hollow channel/the microfluidic channel network. The method may also include that either during cultivation or in the use of the cultivated cells with the method of the present invention, capturing a plurality of images of the cells, gel, and/or microfluidic channel networks in the microfluidic culture system.

Also provided is that simultaneously with or after any of steps a) - d) the cells are contacted with a test compound. The test compound may be any type of compound, for example a drug, a material found in food or in blood. It is even contemplated the test compound is a bacteria, virus of eukaryotic cell (including e.g. blood cells). The effect of such compound on epithelial function may be determined by comparison to conditions in the absence of such compound.

As the skilled person understand, the cells obtained in the microfluidic system with the method of the present invention may be used in a wide variety of settings. For example for assessing transport over the epithelial barrier, toxicity studies, co-culture with microbiome, food absorption studies, inflammation studies, providing disease models, such as inflammatory bowel disease, cystic fibrosis, COPD, asthma, cancer, for mechanistic studies on epithelial function in healthy and diseased conditions, and the like. The skilled person understands how to use the cells cultivated according to the present invention within the context of such experimental settings. Using the microfluidic systems in accordance with the present invention allows for reliable high-throughput testing.

Also provided is a composition or system comprising a microfluidic cell culture system with a microfluidic channel network comprising an inner group of cells and an outer group of cells, wherein the inner group of cells is at least partially covered by said outer group of cells and wherein the cells of the inner group are epithelial cells and the cells of the outer group are mesenchymal cells, preferably wherein the inner group of cells and the outer group of cell interact or are in direct contact. In other words, also provided is a microfluidic cell culture device comprising therein a layer of mesenchymal cells and a layer of epithelial cells, in close contact with each other and as described herein. Preferably the mesenchymal cells and the epithelial cells are in the form of a tubular structure as defined herein.

Also, there is provided for a method of culturing and/or monitoring epithelial cells using a microfluidic cell culture system comprising a microfluidic channel network, the method comprising
a) introducing a mixture of epithelial and mesenchymal cells in the microfluidic channel network, wherein the mixture of cells is introduced in the microfluidic channel network using an aqueous medium;
b) allowing the mesenchymal cells and the epithelial cells to proliferate, preferably until at least part of the microfluidic channel network is covered with cells.

Finally, there is provided for a microfluidic cell culture system comprising mesenchymal cells and epithelial cells, preferably wherein the mesenchymal cells and epithelial cells form a tubular structure or for a microfluidic cell culture system comprising mesenchymal cells and epithelial cells obtainable by the method of culturing and/or monitoring epithelial cells of the present invention.

It will be understood by the skilled person that such microfluidic cell culture system with the mesenchymal cells and epithelial cells provides important advantages. With such system, consumers can, for example, be provided with ready to go systems (e.g. for testing), already comprising the appropriate cells, or with systems than only require limited further cultivation and handling. This improves reproducibility and quality of the experimental data obtained when using the cells cultivated with the method of the invention. It will be understood that the microfluidic cell culture system may thus comprise the mesenchymal and epithelial cells that may be at any developmental stage as described herein.

The skilled person understands that with respect to the various embodiments and preference with respect to this method reference can be made to the various embodiments and preferences described herein throughout the description and claims, as far as applicable to this method.

Having now generally described the invention, the same will be more readily understood through reference to the following examples which is provided by way of illustration and is not intended to be limiting of the present invention.

### Examples

### Example 1

### Materials and Methods

Hedgehog, Wnt and BMP signals may be required during developmental patterning of the intestinal tract as well as for establishing the crypt-villus axis. In vivo, intestinal epithelial cells interact and relay on the signals from underlying mesenchyme. Intestinal mesenchymal cells dynamically contribute in epithelial-mesenchymal interactions, regulating both epithelial proliferation and differentiation.

To establish the crypt-villus axis in the microfluidic model of intestinal tract we made use of the intestinal organoid cultures that were established from human intestinal tissue samples as described (Sato, T. et al., 2011, Gastroenterol). Organoids from mouse, canine, feline etc may also be used.

Organoids were embedded in 10-50 microl ECM (e.g. Matrigel, preferably matrigel, BME (Cultrex Basement Membrane Extracts, BME2) seeded in 48-, or 24-wellplate and overlaid with 250-750 microliter of **basal medium** composed of advanced Dulbecco's modified Eagle medium/F12 supplemented with penicillin/streptomycin, 1 x Glutamax, 10 mmol/L HEPES, 1× N2, 1× B27 (all from Life Technologies), 50 ng/ml murine EGF, 1 mmol/L N-acetylcysteine (Sigma), 100 ng/ml murine noggin, 1 µg/ml human R-spondin-1, 1 mM gastrin, 10 mM nicotinamide, 10 µM SB202190, 500 nM A83-01, 50% Wnt3a conditioned medium or 200 (300, 400, 500 or more) ng/ml recombinant Wnt3a protein (R&D). The entire medium was changed every 2-3 days and organoids were passaged 1:2 (or 1:3, 1:4, 1:5) every week.

To model intestinal tract development we used mesenchymal cells, preferably of the intestinal tract origin (for example, mouse embryonic fibroblasts, mouse fibroblasts, human fibroblasts, intestinal fibroblasts, smooth muscle cells, intestinal myo-fibroblasts, preferably of human) and seeded in the 2-lane or 3-lane in the gel (which may for example be collagen I, IV, Hystem c, matrigel) at the density of 1E6 or 5E6 or 10E6 or 15E6 or 20E6 cells/ml in the ECM, or are seeded in the combination of ECM with medium composed of 10 or 15 % FCS in DMEM (or EMEM or RPMI medium) supplemented with pen/strep, 1x NEAA, 1×Glutamax. The ratio between ECM and medium may be, for example, 9:1 or 8:1 or 7:1 or 6:1 or 5:1 or 3:1 or 2:1 or 1:1.

In another experiment the mesenchymal cells, for example of the intestinal tract origin (intestinal fibroblasts, intestinal myo-fibroblasts, preferably of human), are introduced against the ECM. After about 0 - 72 hours, or more, of incubation of the mesenchymal cells, epithelial intestinal organoid cells are introduced to the adjacent channel to the mesenchymal cells.

Patterning of the underlying mesenchyme may be important for patterning of the epithelial cells and crypt formation. During development of the intestinal tract the mesenchymal cells are concentrated in pericryptal regions that will provide cues for crypt formation in the intestinal epithelium. One of the main factors produced by mesenchymal concentrated cells that aid crypt formations are Wnt proteins.

It was found that the intestinal mesenchymal cells can be mobilized to form concentrated cell clusters by providing chemotactic signals such as TGFβ, endothelin 1, PDGF-B, PDGFA and Hedgehog proteins (Shh, Ihh, Hh). The intestinal mesenchymal produces many different types of Wnt proteins.

In one experiment the method mesenchymal cells may be seeded into a gel containing resin soaked with one of combination of these cues (for example Affi-gel beads (Bio Rad, 153-7302) were soaked in hrSHH (for example 0,1 to 1 mg/mL in PBS; R&D Systems; 1845-SH) and seeded with mesenchymal cells in the gel to induce cell concentrations. Next the intestinal organoid cell (single cells or 2-5 cell clusters of cells prepared by using TrypLE for 5'min) were introduced in the next channel in the ECM, or against the ECM. Cells may be seeded at the density of 1E6 or 5E6 or 10E6 or 15E6 or 20E6 cells/ml.

In a 3-lane design of the microfluidic culture device with one type of ECM in the middle lane, mesenchymal cells may be introduced in the gel with concentrating cues on beads (agarose beads are soaked with chemoattractant/ signalling molecule). Next epithelial cells are introduced in one of the adjacent channels.

Polarized epithelial cells rely on the cell-cell contact and when disrupted undergo apoptosis. Therefore, it may be important to provide Rock kinase inhibitors during and after dissociation of epithelial cells to increase their survival. Preferably 10µM Y27632 Rock inhibitor can be used.

Epithelial cells are initially maintained in the basal medium apically and basally for, for example, 1 or 2 or 3 or 4 days. Then the medium in the intestinal epithelial cells channel was depleted of Wnt3a, whereas in the distal channel medium was supplemented with extra wnt3a protein. This might be especially advantageous when culturing intestinal stem cells derived epithelial structures because Wnt proteins will provide signal for maintaining crypt-like structures on one side of the tube, and diminished concentrations ofWnts in the other channel will support differentiation of the epithelial barrier.

Recreating, in the device, the cellular microenvironment and signaling gradients of e.g. Wnt signals found in vivo for intestine was found advantageous for the assembly of functional intestinal tissue. For example, Wnt3a recombinant protein at the concentration at least 100 ng/ml or more is a preferred to be used for the creating gradient of this signal. To amplify the effect that treatment the same gradient should be created with R-spondin (e.g. R-spondin 1) protein at the concentration of, for example, 50 ng/ml of more. Wnt3a conditioned medium and R-spondin 1 condition medium can be also used to create such gradient. The concentrations for R-spondin conditioned medium may preferably be 10% or more. The concentrations for Wnt3a conditioned medium may preferably be 50% or more and preferably not less than 30%.

GSKβ inhibitor small molecule CHIR activates canonical Wnt pathway. CHIR molecule might substitute use of Wnt3a or R-spondin1 proteins during the initial expansion phase of intestinal epithelium in the device, for example when used at the concentration of 3 µM or more. CHIR molecule may not be desired for the creation of a Wnt signalling gradient, since this small molecule may diffuse fast in the culture in contrast to proteins. Another GSKβ inhibitor LiCl at the concentration of, for example, 1mM up to 30mM may be used instead of CHIR.

Bmp signal molecules (e.g. BMP4) are produced and released by underlying mesenchyme in vivo. BMP signaling provide differentiation signal for the intestinal stem cells. It may thus be advantegous to recreate the gradient of BMP inhibitors (e.g. Noggin) to support active proliferation of the stem cell compartment (which is inhibited by BMP) and allow segregation and differentiation of intestinal tract similar to counterparts found in vivo.

Noggin containing medium could be provided on one side of the device, for example, fed at the "bottom" of the crypts. Gradients of this signals may, for example, be created after epithelial cells reached confluency (or before). Medium depleted from Noggin may be provided at the apical side of the engineered tube. This method might be particularly beneficial for maturation of the intestinal lining when specific manifestations of that differentiated state are desired like production of mucins at the apical side.

EGF may also be important for maintaining intestinal stem and progenitor cells in vivo and in vitro. Additionally, when supplemented apically (e.g. with breast milk) it may protect from apoptosis and necrosis of developing intestine in new-boms. Thus EGF supplementation, for example, at the concentration of not less than 10 ng/ml and not more than 100 ng/ml, preferably 50ng/ml, may be kept throughout the culture period to support proliferation of epithelial cells and inhibit apoptosis in these cells.

Notch pathway activity is important for proliferative state of intestinal epithelium and when inhibited with for example γ-secretase inhibitors it may result in terminal differentiation of the intestinal tissue to for example goblet cells. It may thus be beneficial to give a short term pulse of Notch pathway inhibitors to enhance goblet cells maturation for production of mucins. γ-secretase inhibitor (e.g. 10 µM DAPT) may be preferred to be used after initial proliferation of the epithelial cells in the device.

After for example 3 days post epithelial cells seeding or after the epithelial layer of cells reached confluency γ-secretase inhibitor may be added to the apical side medium to induce growth arrest and maturation of the goblet cells. Medium may be depleted of γ-secretase inhibitor to prevent loss of stem cell niche (crypt), preferably within, for example, 5 days of continuous culture in the presence of γ-secretase inhibitor (e.g. after 12h or 24 or 48h and so on). This treatment may improve mucous layer production by mature goblet cells while short treatment with Notch inhibitor and strong Wnt agonists treatment from the basal side (closer to crypt) may ensure that the stem cell niche will be preserved. This subsequent seeding of two cell types followed by periods of treatment with agonists and inhibitors of critical pathways will ensure successful development of mature tubular intestinal mini-organ.

### Example 2- Sequential seeding of mesenchymal and epithelial cells

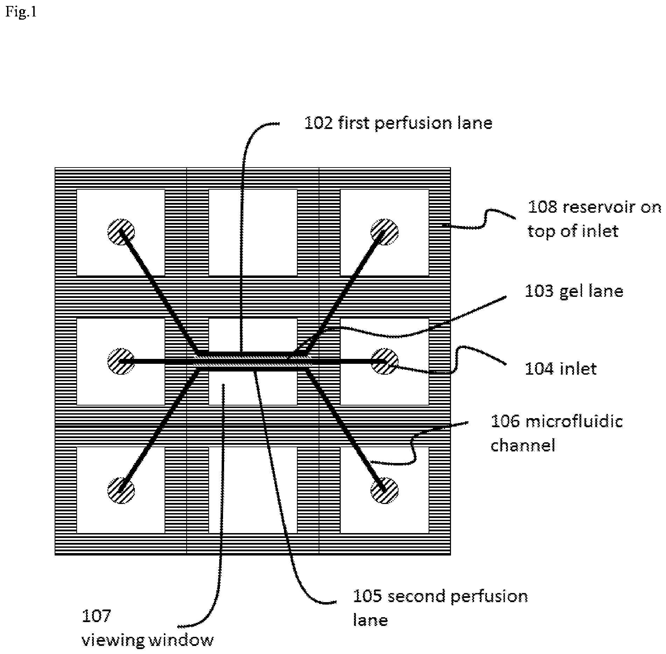

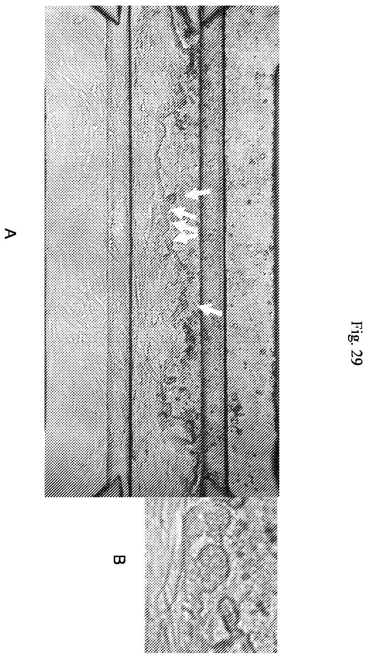

For this experiment a 3-lane OrganoPlate^{®} (MIMETAS) with 400 micron wide lanes as shown in figure 1 was used. Intestinal myofibroblasts, seeded in an ECM gel (see below), in a concentration of 5000 cells/experiment, were injected in the gel lane (103). Thereafter , CaCO-2 cells in EMEM medium (as described below) were injected in the perfusion lane (102) in a concentration of 20,000 cells/experiment. Next, the Caco-2 cells were cultivated for 7 days (in the presence of the myofibroblasts). In the third microfluidic channel (106) smGM medium (smooth muscle growth medium; Lonza) was present. On the 7^{th} day, phase contrast images were taken, the result of which is shown in figure 29A and 29B.

It can be seen from these figures that the Caco-2 cells entered the gel lane containing the myfibroblasts, interacting with the myofibroblasts and forming a layer on top. In addition the experiment show that secondary morphology and organization is formed where the Caco-2 cells and myofibroblasts are interacting. Arrows point at such structures that look similar to, for example, crypt or villi morphology found in the colon or small intestine, and closely resembling the in vivo situation.

### EMEM medium:

EMEM (ATCC, Cat.No. 30-2003)
Pen/Strep 1% (Sigma, Cat.No. P4333)
MEM Non-Essential Amino Acids Solution (100X) 1% (Gibco, Cat.No. 11140-050)
FBS HI 10% (Gibco, Cat. No. 16140-071).

### ECM gel:

Collagen I 5 mg/mL (AMSbio Cultrex 3D collagen I rat tail, 5 mg/mL, #3447-020-01)
i. 1M HEPES (Life Technologies 15630-122)
ii. 37g/L NaHCO3 (Sigma S5761-500G))

### SmGM-2 Smooth Muscle Growth Medium-2

### SmGM-2 complete medium:

- SmBM Basal Medium (Lonza, CC-3156)
- SmGM^{™}-2 SingleQuots^{™} Supplements and growth factors (hEGF, insulin, FGF-B, FBS and gentamicin/amphotericinB)

### Example 3: Mesenchymal/epithelial cells tubes

For this experiment a 2-lane OrganoPlate^{®} (MIMETAS) with 400 micron wide lanes was used.

Cells, a 4:1 mixture of vvHUVEC-RFP endothelium cells (Angiocrine, cell passage 4) in Endothelial Cell Growth Medium MV2, (Promocel, Cat: C-22022); and brain vascular pericytes (Sciencell, cell passage 4) in Pericyte Medium (Sciencell); in a total starting concentration of 5000 cells/µL were cultivated while placing the 2-lane OrganoPlate^{®} on a perfusion rocker (7° inclination angle, 8 min rocking cycle).

After 3 days of culturing, the cells were stained using Actin-Green. Images of the formed tube were made using confocal microscopy (Leica, TCS SP5 STED). 3D projection was created using the 3D viewer Fiji plug in (Schindelin, J.; Arganda-Carreras, I. & Frise, E. et al. (2012), "Fiji: an open-source platform for biological-image analysis", Nature methods 9(7): 676-682, PMID 22743772.). Results are shown in fig. 31 As can be seen, the mesenchymal cells and endothelial cells are able to form a tube comprising both endothelial cells and pericytes .

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

1. Method of culturing and/or monitoring epithelial cells using a microfluidic cell culture system comprising a microfluidic channel network, the method comprising
a) introducing mesenchymal cells in the microfluidic channel network, wherein the mesenchymal cells are introduced in the microfluidic channel network
a1) using an aqueous medium; or
a2) using a gel precursor and allowing the gel precursor to gelate in the microfluidic channel network thereby occupying at least part of the microfluidic channel network;
b) in case of step a1), and preferably in case of step a2), allowing the mesenchymal cells to proliferate and/or differentiate, until at least part of the microfluidic channel network is covered with mesenchymal cells such that at least a group/layer/sheet of mesenchymal cells is formed;
c) introducing epithelial cells in the microfluidic channel network comprising the mesenchymal cells; and
d) allowing the epithelial cells to proliferate and/or differentiate, until at least part of the microfluidic channel network is covered with epithelial cells and/or until at least part of the mesenchymal cells is covered with epithelial cells such that at least a group/layer/sheet of epithelial cells is formed.

2. Method according to claim 1, wherein a gel precursor is introduced in the microfluidic channel network and allowing the gel precursor to gelate in the microfluidic channel network thereby occupying at least part of the microfluidic channel network, preferably wherein the gel is patterned, more preferably by use of a capillary pressure barrier, by UV patterning, or by retracting a needle after gelation, or by having a sacrificial layer that is removed after gelation.

3. Method of any of the previous claims wherein in step b) the mesenchymal cells are proliferated and/or differentiated until at least a tubular structure of mesenchymal cells is formed in the microfluidic channel network; and optionally
wherein in step d) the epithelial cells are proliferated and/or differentiated until at least a tubular structure of epithelial cells is formed in the microfluidic channel network.

4. Method of any of the previous claims wherein a flow of growth medium through the lumen of the tubular structure is applied, wherein said flow may be uni-directional or bidirectional.

5. Method of any of the previous claims wherein in step d) the epithelial cells are allowed to form a layer of cells with an apical and a basolateral side, the basolateral side being faced towards the mesenchymal cells, preferably wherein at least part of the mesenchymal cells are in direct contact with at least part of the epithelial cells and/or wherein the distance between the mesenchymal cell sheet and the epithelial cell sheet is the thickness or less than the thickness of a basal lamina.

6. Method of any of the previous claims wherein the mesenchymal cells are selected from myofibroblasts, fibroblasts, adipocytes, chondroblasts, osteoblasts, smooth muscle cells and stromal cells, preferably wherein the mesenchymal cells are mammalian cells, preferably human cells, preferably wherein the mesenchymal cells are primary cells; and/or wherein the epithelial cells are selected from simple epithelia cells, simple squamous epithelia cells, stratified epithelia cells, or columnar epithelia cells, preferably wherein the epithelial cells are mammalian cells, preferably human cells, preferably wherein the epithelial cells are primary cells.

7. Method of any of the previous claims wherein the microfluidic cell culture system comprises a culture chamber, wherein the mesenchymal cells in step a) and the epithelial cells in step c) are introduced.

8. Method of any of the previous claims wherein in step a) different types of mesenchymal cells are introduced and/or wherein in step c) different types of epithelial cells are introduced in the same microfluidic channel.

9. Method of any of the previous claims wherein the microfluidic cell culture system provides an uninterrupted optical path to the cells in the microfluidic channel network and/or to the gel and/or to the further microfluidic channel network.

10. Method of any of the previous claims wherein simultaneously with or after any of steps a) - d) the cells are contacted with a test compound.

11. Method of any of the previous claims wherein the method is for assessing transport over the epithelial barrier, toxicity studies, co-culture with microbiome, food absorption studies, inflammation studies, providing disease models, such as inflammatory bowel disease, cystic fibrosis, COPD, asthma, cancer, for mechanistic studies on epithelial function in healthy and diseased conditions.

## Patentansprüche

1. Verfahren zum Kultivieren und/oder Überwachen von Epithelzellen unter Einsatz eines Mikrofluidikzellkultursystems, umfassend ein Mikrofluidikkanalnetzwerk, das Verfahren umfassend:
a) Einführen von mesenchymalen Zellen in das Mikrofluidikkanalnetzwerk, wobei die mesenchymalen Zellen in das Mikrofluidikkanalnetzwerk eingeführt werden:
a1) unter Einsatz eines wässrigen Mediums; oder
a2) unter Einsatz eines Gelpräkursors, wobei ermöglicht wird, dass der Gelpräkursor in dem Mikrofluidikkanalnetzwerk geliert, wodurch mindestens ein Teil des Mikrofluidikkanalnetzwerks belegt wird;
b) im Falle von Schritt a1) und vorzugsweise im Falle von Schritt a2), Ermöglichen, dass die mesenchymalen Zellen proliferieren und/oder differenzieren, bis mindestens ein Teil des Mikrofluidikkanalnetzwerks mit mesenchymalen Zellen bedeckt ist, sodass mindestens eine Gruppe/Schicht/Lage von mesenchymalen Zellen ausgebildet wird;
c) Einführen von Epithelzellen in das Mikrofluidikkanalnetzwerk, umfassend die mesenchymalen Zellen; und
d) Ermöglichen, dass die Epithelzellen proliferieren und/oder differenzieren, bis mindestens ein Teil des Mikrofluidikkanalnetzwerks mit Epithelzellen bedeckt ist und/oder bis mindestens ein Teil der mesenchymalen Zellen mit Epithelzellen bedeckt ist, sodass mindestens eine Gruppe/Schicht/Lage von mesenchymalen Zellen ausgebildet wird.

2. Verfahren nach Anspruch 1, wobei ein Gelpräkursor in das Mikrofluidikkanalnetzwerk eingeführt wird und es ermöglicht wird, dass das der Gelpräkursor in dem Mikrofluidikkanalnetzwerk geliert, wodurch mindestens ein Teil des Mikrofluidikkanalnetzwerk bedeckt wird, vorzugsweise wobei das Gel strukturiert ist, stärker bevorzugt unter Einsatz einer Kapillardruckbarriere, durch UV-Strukturierung oder durch Zurückziehen einer Nadel nach dem Gelieren oder durch Einsatz einer Opferschicht, die nach dem Gelieren entfernt wird.

3. Verfahren nach einem der vorhergehenden Ansprüche, wobei in Schritt b die mesenchymalen Zellen proliferiert und/oder differenziert werden, bis in dem Mikrofluidikkanalnetzwerk mindestens eine rohrförmige Struktur aus mesenchymalen Zellen ausgebildet wird; und optional
wobei in Schritt d) die Epithelzellen proliferiert und/oder differenziert werden, bis mindestens eine rohrförmige Struktur von Epithelzellen in dem Mikrofluidikkanalnetzwerk ausgebildet wird.

4. Verfahren nach einem der vorhergehenden Ansprüche, wobei ein Strom von Wachstumsmedium durch das Lumen der rohrförmigen Struktur angelegt wird,wobei der Strom unidirektional oder bidirektional sein kann.

5. Verfahren nach einem der vorhergehenden Ansprüche, wobei es in Schritt d) den Epithelzellen ermöglicht wird, eine Schicht aus Zellen mit einer apikalen und einer basolaterlaen Seite auszubilden, wobei die basolaterale Seite zu den mesenchymalen Zellen hin weist, vorzugsweise wobei mindestens ein Teil der mesenchymalen Zellen in direktem Kontakt mit mindestens einem Teil der Epithelzellen steht und/oder wobei der Abstand zwischen der mesenchymalen Zelllage und der Epithelzelllage höchstens der Dicke einer Basalmembran entspricht.

6. Verfahren nach einem der vorhergehenden Ansprüche, wobei die mesenchymalen Zellen ausgewählt sind aus Myofibroblasten, Fibroblasten, Adipozyten, Chondroblasten, Osteoblasten, glatten Muskelzellen und Stromazellen, wobei die mesenchymalen Zellen vorzugsweise Säugerzellen sind, vorzugsweise menschliche Zellen, vorzugsweise wobei die mesenchymalen Zellen primäre Zellen sind; und/oder wobei die Epithelzellen ausgewählt sind aus einfachen Epithelzellen, einfachen Plattenepithelzellen, mehrschichtigen Epithelzellen und säulenförmigen Epithelzellen, vorzugsweise wobei die Epithelzellen Säugerzellen sind, vorzugsweise menschliche Zellen, vorzugsweise wobei die Epithelzellen primäre Zellen sind.

7. Verfahren nach einem der vorhergehenden Ansprüche, wobei das Mikrofluidikzellkultursystem eine Kulturkammer umfasst, wobei die mesenchymalen Zellen in Schritt a) und die Epithelzellen in Schritt c) eingeführt werden.

8. Verfahren nach einem der vorhergehenden Ansprüche, wobei im gleichen Mikrofluidikkanal in Schritt a) verschiedene Arten von mesenchymalen Zellen eingeführt werden und/oder in Schritt c) verschiedene Arten von Epithelzellen eingeführt werden.

9. Verfahren nach einem der vorhergehenden Ansprüche, wobei das Mikrofluidikzellkultursystem einen ununterbrochenen optischen Pfad zu den Zellen in dem Mikrofluidikkanalnetzwerk und/oder zu dem Gel und/oder zu dem weiteren Mikrofluidikkanalnetzwerk bereitstellt.

10. Verfahren nach einem der vorhergehenden Ansprüche, wobei gleichzeitig mit oder nach beliebigen der Schritte a)-d) die Zellen mit einer Testverbindung in Berührung gebracht werden.

11. Verfahren nach einem der vorhergehenden Ansprüche, wobei das Verfahren vorgesehen ist zum Auswerten des Transports über die Epithelbarriere, für Toxizitätsstudien, zum Co-Kultivieren mit einem Mikrobiom, für Lebensmittelabsorptionsstudien, für Entzündungsstudien, zum Bereitstellen von Erkrankungsmodellen, wie etwa für entzündliche Darmerkrankung, Mukoviszidose, COPD, Asthma, Krebs sowie für mechanistische Studien der Epithelfunktion im gesunden sowie im erkrankten Zustand.

## Revendications

1. Procédé de culture et/ou de surveillance de cellules épithéliales en utilisant un système de culture cellulaire microfluidique comprenant un réseau de canaux microfluidiques, le procédé comprenant
a) l'introduction des cellules mésenchymateuses dans le réseau de canaux microfluidiques, dans lequel les cellules mésenchymateuses sont introduites dans le réseau de canaux microfluidiques
a1) en utilisant un milieu aqueux ; ou
a2) en utilisant un précurseur de gel et en permettant au précurseur de gel de gélifier dans le réseau de canaux microfluidiques, occupant ainsi au moins une partie du réseau de canaux microfluidiques ;
b) dans le cas de l'étape a1), et de préférence dans le cas de l'étape a2), la permission aux cellules mésenchymateuses de proliférer et/ou de différencier, jusqu'à ce qu'au moins une partie du réseau de canaux microfluidiques soit recouverte de cellules mésenchymateuses de sorte qu'au moins un groupe/une couche/une feuille de cellules mésenchymateuses soit formé(e) ;
c) l'introduction des cellules épithéliales dans le réseau de canaux microfluidiques comprenant les cellules mésenchymateuses ; et
d) la permission aux cellules épithéliales de proliférer et/ou de différencier, jusqu'à ce qu'au moins une partie du réseau de canaux microfluidiques soit recouverte de cellules épithéliales et/ou jusqu'à ce qu'au moins une partie des cellules mésenchymateuses soit recouverte de cellules épithéliales de sorte qu'au moins un groupe/une couche/une feuille de cellules épithéliales soit formé(e).

2. Procédé selon la revendication 1, dans lequel un précurseur de gel est introduit dans le réseau de canaux microfluidiques et permettant au précurseur de gel de gélifier dans le réseau de canaux microfluidiques occupant ainsi au moins une partie du réseau de canaux microfluidiques, de préférence dans lequel le gel est structuré, plus préférablement en utilisant une barrière de pression capillaire, par structuration UV, ou en rétractant une aiguille après gélification, ou en ayant une couche sacrificielle qui est retirée après gélification.

3. Procédé de l'une des revendications précédentes dans lequel à l'étape b) les cellules mésenchymateuses prolifèrent et/ou sont différenciées jusqu'à ce qu'au moins une structure tubulaire de cellules mésenchymateuses soit formée dans le réseau de canaux microfluidiques ; et éventuellement
dans lequel, à l'étape d), les cellules épithéliales prolifèrent et/ou sont différenciées jusqu'à ce qu'au moins une structure tubulaire de cellules épithéliales soit formée dans le réseau de canaux microfluidiques.

4. Procédé de l'une des revendications précédentes dans lequel un écoulement de milieu de culture à travers la lumière de la structure tubulaire est appliqué, dans lequel ledit écoulement peut être unidirectionnel ou bidirectionnel.

5. Procédé de l'une des revendications précédentes, dans lequel à l'étape d) les cellules épithéliales sont autorisées à former une couche de cellules avec un côté apical et un côté basolatéral, le côté basolatéral étant tourné vers les cellules mésenchymateuses, de préférence dans lequel au moins une partie des cellules mésenchymateuses sont en contact direct avec au moins une partie des cellules épithéliales et/ou dans lequel la distance entre la feuille de cellules mésenchymateuses et la feuille de cellules épithéliales est égale ou inférieure à l'épaisseur d'une lame basale.

6. Procédé de l'une des revendications précédentes, dans lequel les cellules mésenchymateuses sont choisies parmi les myofibroblastes, les fibroblastes, les adipocytes, les chondroblastes, les ostéoblastes, les cellules musculaires lisses et les cellules stromales, de préférence dans lequel les cellules mésenchymateuses sont des cellules de mammifères, de préférence des cellules humaines, de préférence dans lequel les cellules mésenchymateuses sont des cellules primaires ; et/ou dans lequel les cellules épithéliales sont choisies parmi des cellules épithéliales simples, des cellules épithéliales squameuses simples, des cellules épithéliales stratifiées ou des cellules épithéliales colonnaires, de préférence dans lequel les cellules épithéliales sont des cellules de mammifères, de préférence des cellules humaines, de préférence dans lequel les cellules épithéliales sont des cellules primaires.

7. Procédé de l'une des revendications précédentes, dans lequel le système de culture cellulaire microfluidique comprend une chambre de culture, dans lequel les cellules mésenchymateuses à l'étape a) et les cellules épithéliales à l'étape c) sont introduites.

8. Procédé de l'une des revendications précédentes dans lequel à l'étape a) différents types de cellules mésenchymateuses sont introduits et/ou dans lequel à l'étape c) différents types de cellules épithéliales sont introduits dans le même canal microfluidique.

9. Procédé de l'une des revendications précédentes, dans lequel le système de culture cellulaire microfluidique fournit un chemin optique ininterrompu vers les cellules dans le réseau de canaux microfluidiques et/ou vers le gel et/ou vers l'autre réseau de canaux microfluidiques.

10. Procédé de l'une des revendications précédentes dans lequel simultanément avec ou après l'une des étapes a) à d), les cellules sont mises en contact avec un composé d'essai.

11. Procédé de l'une des revendications précédentes dans lequel le procédé est destiné à évaluer le transport à travers la barrière épithéliale, les études de toxicité, la co-culture avec le microbiome, les études d'absorption alimentaire, les études d'inflammation, la fourniture de modèles de maladies, tels que la maladie inflammatoire de l'intestin, la mucoviscidose, la BPCO, l'asthme, le cancer, pour des études mécanistiques sur la fonction épithéliale dans des conditions saines et pathologiques.
